# EUROPEAN PATENT SPECIFICATION

(11) **EP 3 747 499 B1**
(45) Date of publication and mention of the grant of the patent: **10.06.2026**
(21) Application number: 19760442.4
(22) Date of filing: 27.02.2019
(51) Int. Cl.: A61M 25/09, A61B 17/34, A61M 25/01, A61M 25/06, A61B 17/00, A61B 90/00

(54) **GUIDE WIRE AND MEDICAL DEVICE**
FÜHRUNGSDRAHT UND MEDIZINISCHE VORRICHTUNG
FIL DE GUIDAGE ET DISPOSITIF MÉDICAL

(30) Priority: 02.03.2018 JP 2018037626
(43) Date of publication of application: 09.12.2020
(73) Proprietor: Terumo Kabushiki Kaisha, Tokyo 151-0072 (JP)
(72) Inventor: HAYAKAWA, Koichi, Ashigarakami-gun, Kanagawa 259-0151 (JP); FUKAMI, Kazunari, Ashigarakami-gun, Kanagawa 259-0151 (JP)
(74) Representative: Casalonga
(86) International application number: PCT/JP2019/007439
(87) International publication number: WO 2019/167985

(56) References cited:
- EP-A1- 2 994 187
- WO-A1-2018/181520
- JP-A- 2007 000 463
- JP-A- 2010 284 501
- JP-A- H08 501 717
- US-A1- 2005 101 984
- US-A1- 2006 074 398
- US-A1- 2006 079 787
- US-A1- 2012 109 079
- US-A1- 2012 109 079

## Description

### Technical Field

The present invention relates to a guide wire and a medical device for puncturing living body tissue.

### Background Art

With a current flowing in a cardiac muscle tissue called an impulse conduction system, the heart repeats contraction and expansion at suitable timing to circulate blood. When the generation and transmission of the electrical signal flowing in the impulse conduction system become abnormal, the contraction and expansion cannot be performed at suitable timing, whereby arrhythmia is generated.

As a therapeutic method for arrhythmia, a method has been known in which a conduction path for a signal that causes arrhythmia is ablated and blocked by heating or cooling. As a device for carrying out this therapeutic method, a device which is percutaneously inserted into the left atrium and by which the conduction path for the signal located at the opening of the pulmonary vein can be ablated has been known. Such an ablation device is frequently used as being low in invasiveness and producing a high effect.

In performing ablation in the left atrium, a technique called atrial septum puncture (Brockenbrough method) is needed in which a needle is made to puncture from the right atrium to a thin partition wall called oval fossa of atrial septum so as to open a hole for communication from the right atrium to the left atrium. Transseptal needles as the device for performing the atrial septum puncture include mechanical needles and radio frequency needles. The mechanical needle is the mainstream because of its inexpensiveness.

The mechanical needle is for puncture by use of a sharp needle. When the mechanical needle is used, there arises a risk of erroneous puncture by pressing the needle excessively. When erroneous puncture by the needle occurs, there is a risk of serious complication called pericardial tamponade (a state in which blood is reserved between pericardium and cardiac muscle to cause cardiac insufficiency). On the other hand, the radio frequency needle is for a method in which high frequency energy supplied from a console that is a device separately provided is outputted to penetrate the atrial septum. Therefore, the radio frequency needle is free of risk of erroneous puncture, but it is expensive and requires the console.

For example, Patent Document 1 describes a device in which an inner needle as a mechanical needle is disposed inside a pipe-shaped outer needle. The tip of the inner needle is curved to be directed to the proximal side. The tip of the inner needle is accommodated in the outer needle in a rectilinearly stretched state. The inner needle, by protruding from the outer needle, opens a hole in the atrial septum from the right atrium side, and, after reaching the left atrium, is bent to be directed toward the proximal side. In this way, the device described in Patent Document 1 restrains generation of erroneous puncture by the inner needle.

### Prior Art Document

### Patent Document

Patent Document 1: U.S. Patent No. 8992556

### Summary of Invention

### Technical Problem

A device for puncturing living body tissue is required to have high workability and safety. The present invention has been made for solving the above-mentioned problem, and it is an object of the present invention to provide a guide wire and a medical device that have high workability, restrain erroneous puncture by a needle portion for puncture, and assure safety.

### Technical Solution

A guide wire according to the present invention for achieving the above object is a guide wire for guiding a pipe-shaped elongate body to be inserted in a living body, the guide wire including a flexible elongate shaft portion, a puncture portion that is disposed at a distal portion of the shaft portion and that forms a hole in living body tissue, and a cover portion that is elastically deformable and that covers the puncture portion, in which when the cover portion is exposed from the elongate body, the cover portion covers the puncture portion, and at least one of the cover portion and the shaft portion is bent.

A medical device according to the present invention for achieving the above object is a medical device for forming a hole in living body tissue of a living body, the medical device including a pipe-shaped elongate body to be inserted in the living body, and a guide wire insertable in the elongate body. The guide wire includes a flexible elongate shaft portion, a puncture portion that is disposed at a distal portion of the shaft portion and that forms the hole in the living body tissue, and a cover portion that is elastically deformable and that covers the puncture portion, in which when the cover portion is exposed from the elongate body, the cover portion covers the puncture portion, and at least one of the cover portion and the shaft portion is bent.

### Advantageous Effect

The guide wire and the medical device configured as above have high workability since they do not need a separate device having a puncturing function. In addition, erroneous puncture by a needle portion for puncture is restrained, and high safety is obtained.

### Brief Description of Drawings

[FIG. 1]

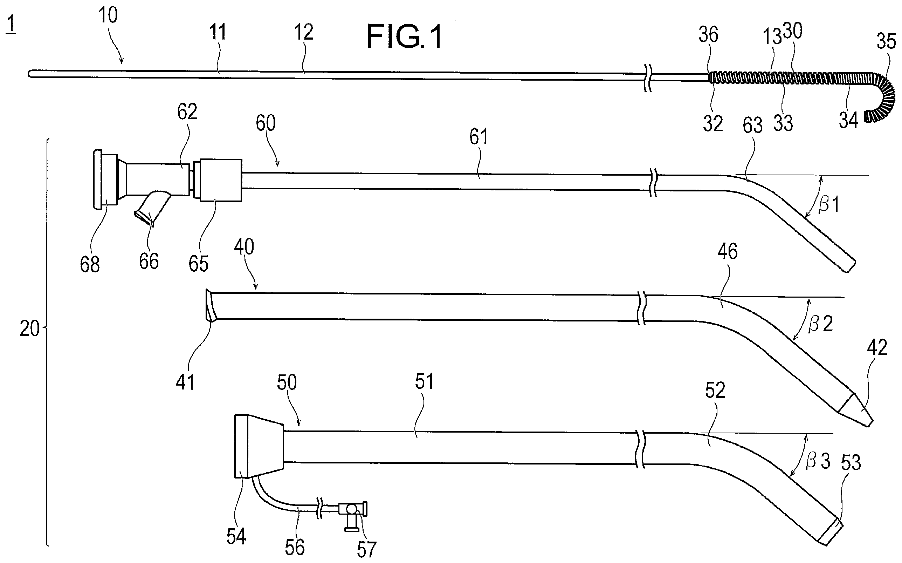

   FIG. 1 is a plan view depicting a medical device according to an embodiment.
[FIG. 2]

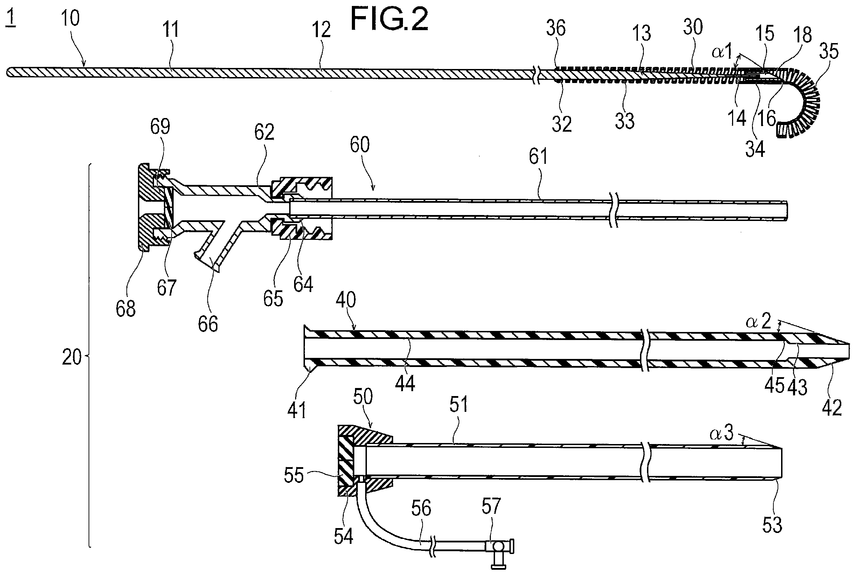

   FIG. 2 is a sectional view depicting the medical device according to the embodiment.
[FIG. 3]

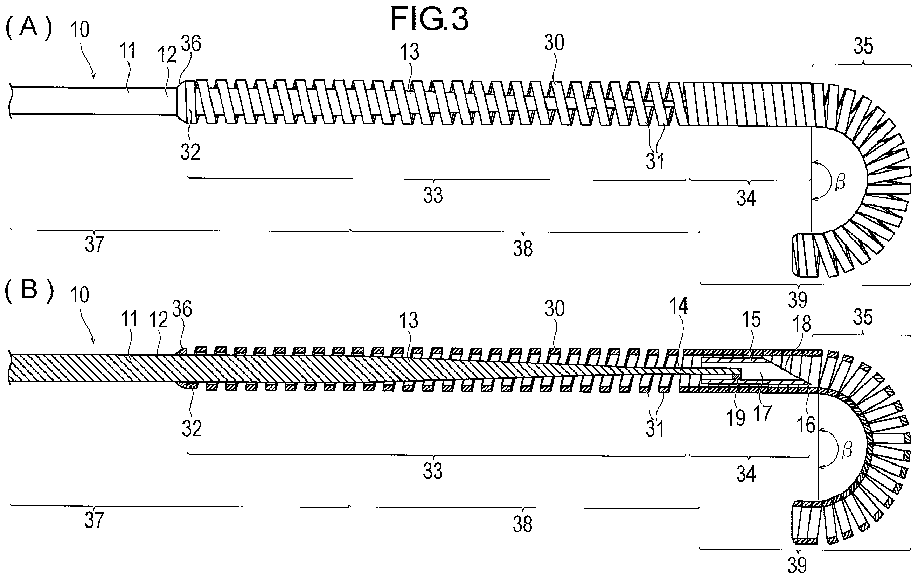

   FIG. 3 illustrates diagrams depicting a guide wire, in which (A) is a plan view and (B) is a sectional view.
[FIG. 4]

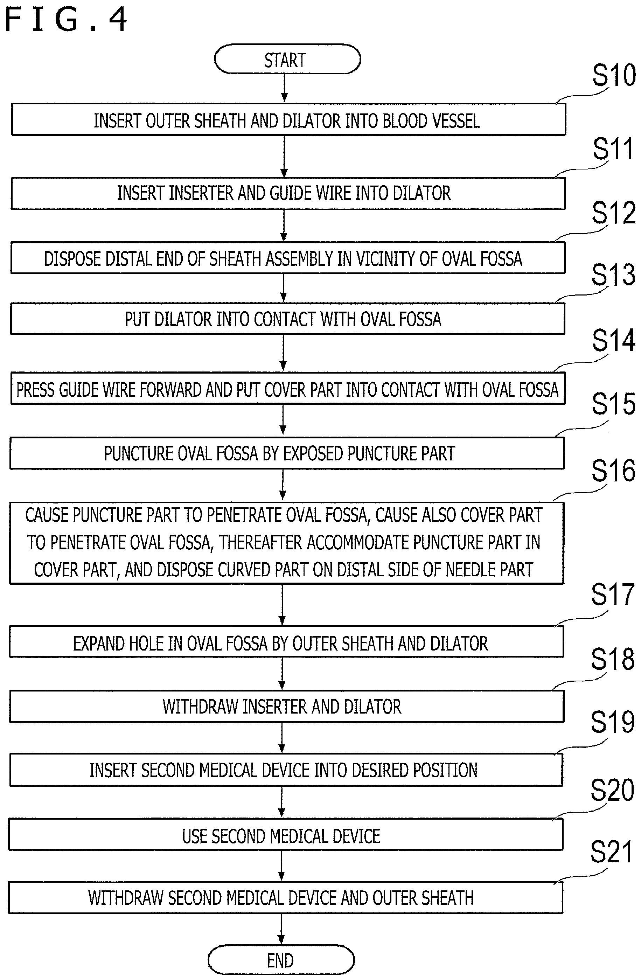

   FIG. 4 is a flow chart for explaining a technique using the medical device.
[FIG. 5]

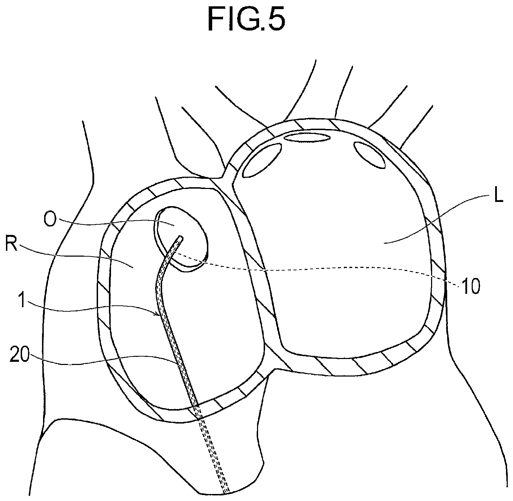

   FIG. 5 is a partial sectional view depicting the inside of a heart.
[FIG. 6]

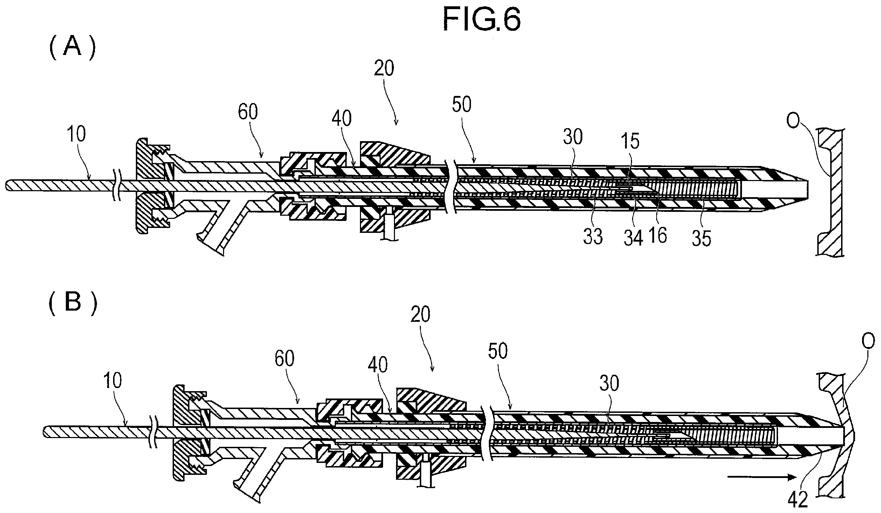

   FIG. 6 illustrates sectional views depicting a state at the time of puncturing by the medical device, in which (A) depicts a state in which a sheath assembly is inserted into a right atrium along the guide wire, and (B) depicts a state in which a dilator is pressed against an oval fossa.
[FIG. 7]

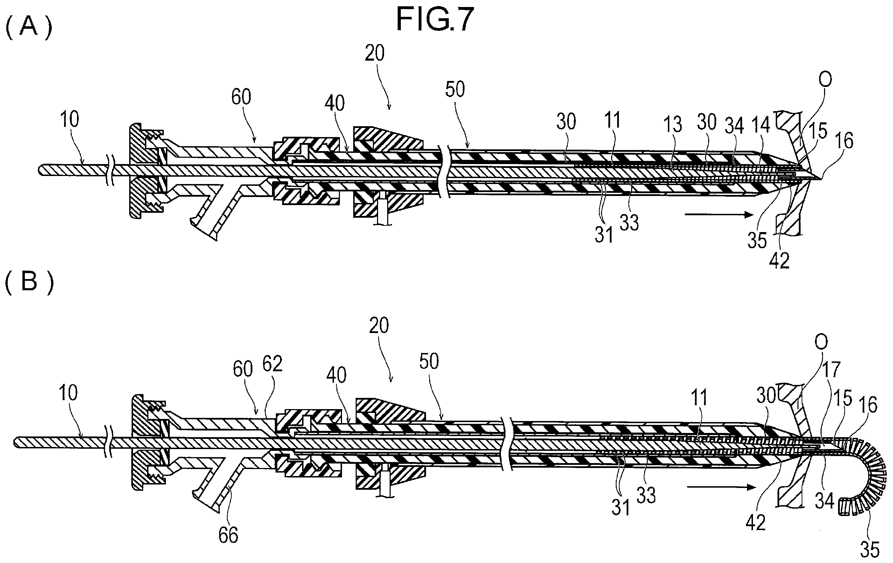

   FIG. 7 illustrates sectional views depicting a state at the time of puncturing by the medical device, in which (A) depicts a state in which the oval fossa is punctured by a puncture portion, and (B) depicts a state in which the puncture portion is accommodated in a cover portion.
[FIG. 8]

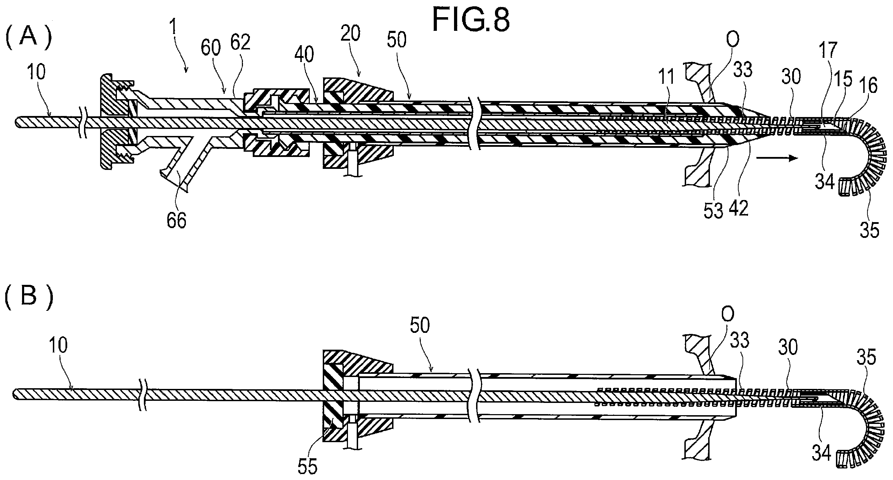

   FIG. 8 illustrates sectional views depicting a state at the time of puncturing by the medical device, in which (A) depicts a state in which the sheath assembly is inserted in a hole of the oval fossa, and (B) depicts a state in which the dilator and an inserter have been drawn out of an outer sheath.
[FIG. 9]

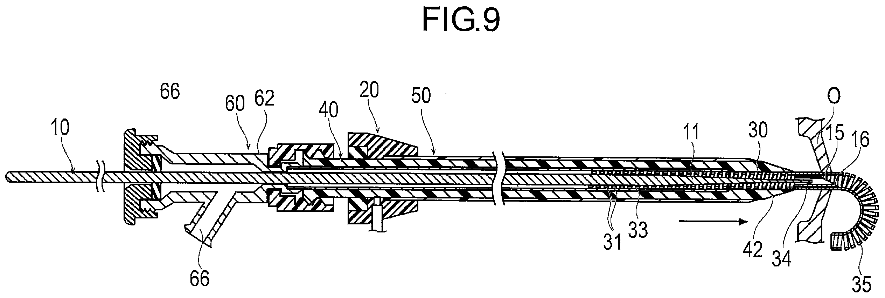

   FIG. 9 is a sectional view depicting a state in which the dilator is separated from the oval fossa after the guide wire is inserted into the hole of the oval fossa.
[FIG. 10]

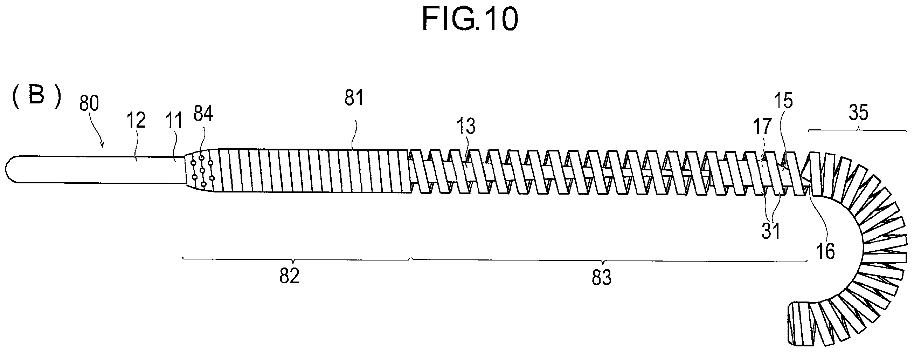

   FIG. 10 is a plan view depicting a first modification.
[FIG. 11]

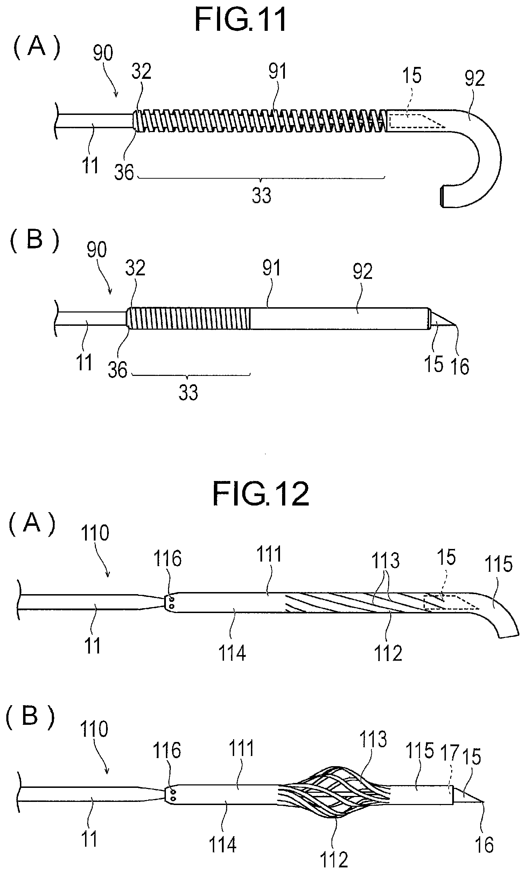

   FIG. 11 illustrates plan views depicting a second modification, in which (A) depicts a state before a cover portion of a guide wire is shrunk, and (B) depicts a state in which a coarse pitch portion of the cover portion of the guide wire has been shrunk.
[FIG. 12]
   FIG. 12 illustrates plan views depicting a third modification, in which (A) depicts a state before a cover portion of a guide wire is shrunk in an axial direction, and (B) depicts a state in which the cover portion of the guide wire has been shrunk in the axial direction.
[FIG. 13]

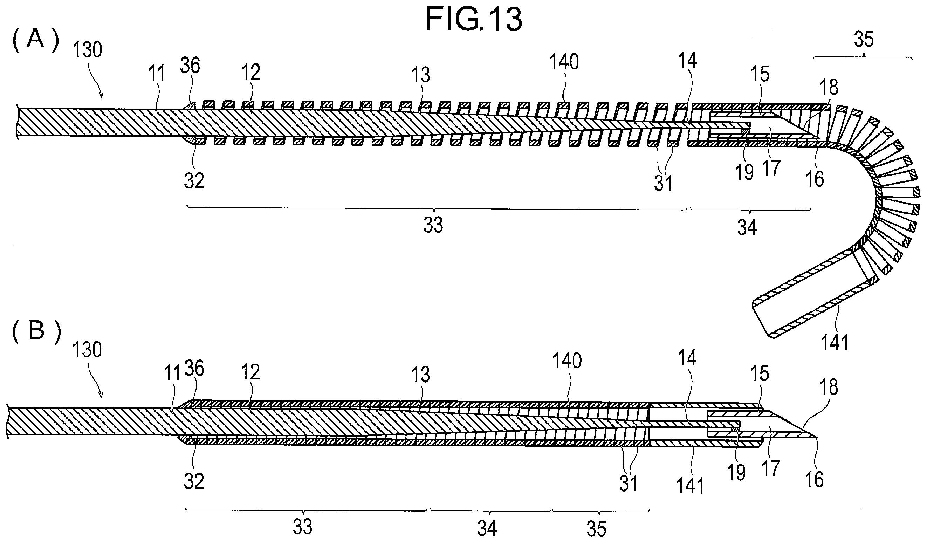

   FIG. 13 illustrates sectional views depicting a fourth modification, in which (A) depicts a state before a cover portion of a guide wire is shrunk, and (B) depicts a state in which a coarse pitch portion of the cover portion of the guide wire has been shrunk.
[FIG. 14]

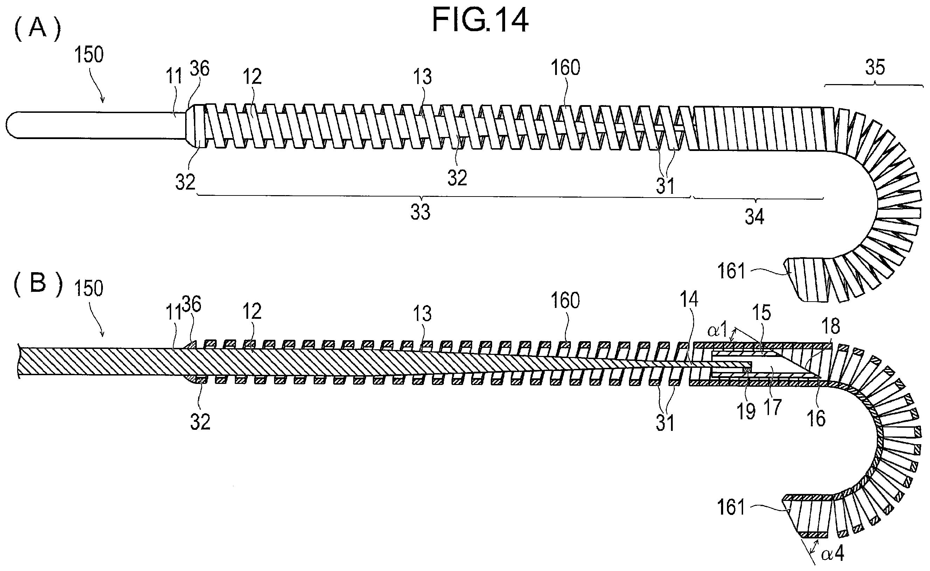

   FIG. 14 illustrates diagrams depicting a guide wire of a fifth modification, in which (A) is a plan view and (B) is a sectional view.
[FIG. 15]

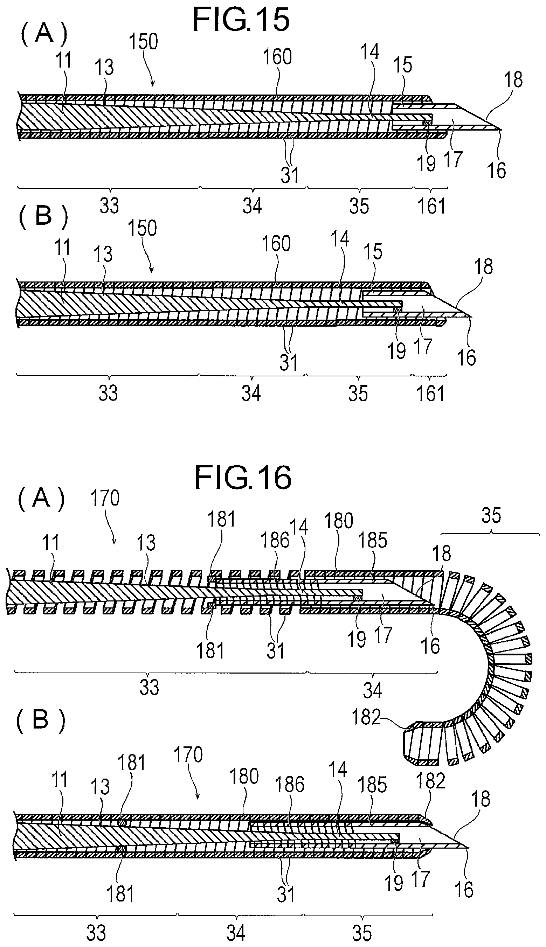

   FIG. 15 illustrates sectional views depicting modifications, in which (A) depicts a state in which a cover portion of the guide wire in the fifth modification has been shrunk in the axial direction, and (B) depicts a state in which the cover portion of the guide wire in a sixth modification has been shrunk in the axial direction.
[FIG. 16]
   FIG. 16 is a sectional view depicting a guide wire of a seventh modification.
[FIG. 17]

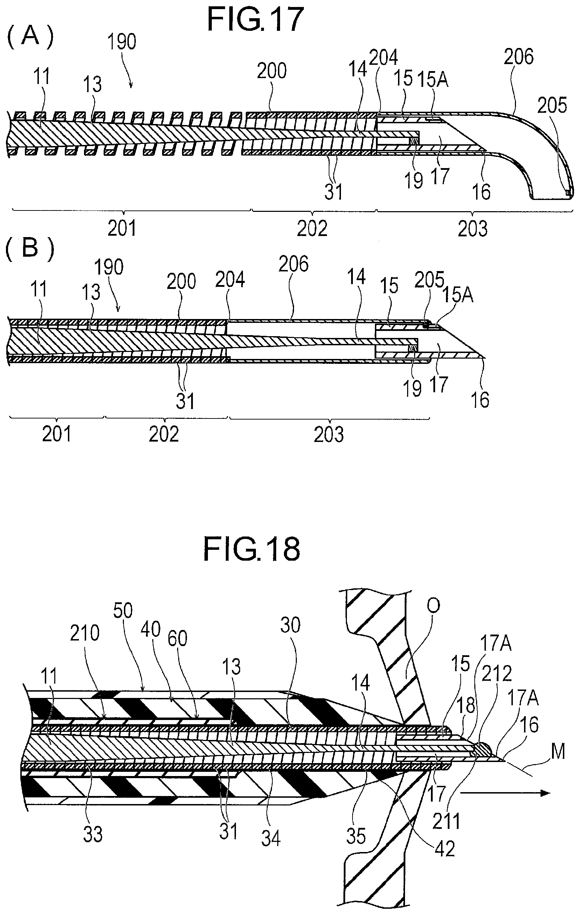

   FIG. 17 illustrates sectional views depicting an eighth modification, in which (A) depicts a state before a cover portion of a guide wire is shrunk in an axial direction, and (B) depicts a state in which the cover portion of the guide wire has been shrunk in the axial direction.
[FIG. 18]
   FIG. 18 is a sectional view depicting a state when puncture is conducted by a ninth modification.
[FIG. 19]

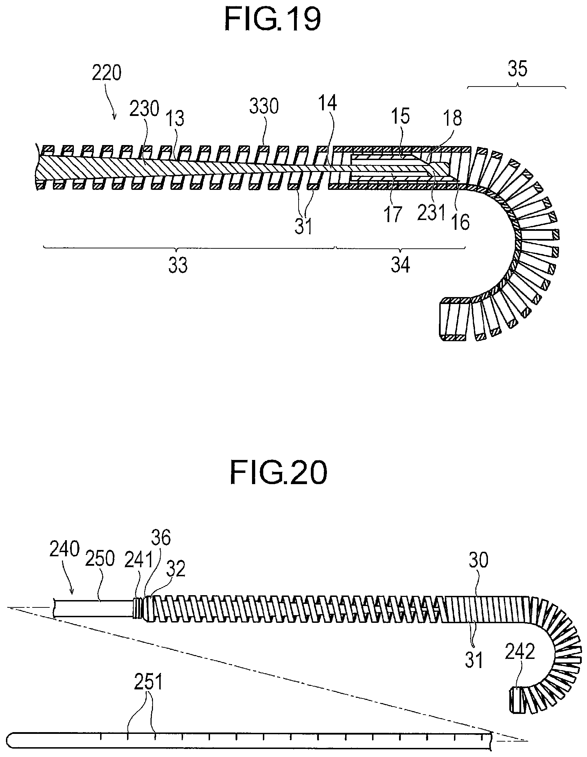

   FIG. 19 is a sectional view depicting a guide wire of a tenth modification.
[FIG. 20]
   FIG. 20 is a plan view depicting a guide wire of an eleventh modification.
[FIG. 21]

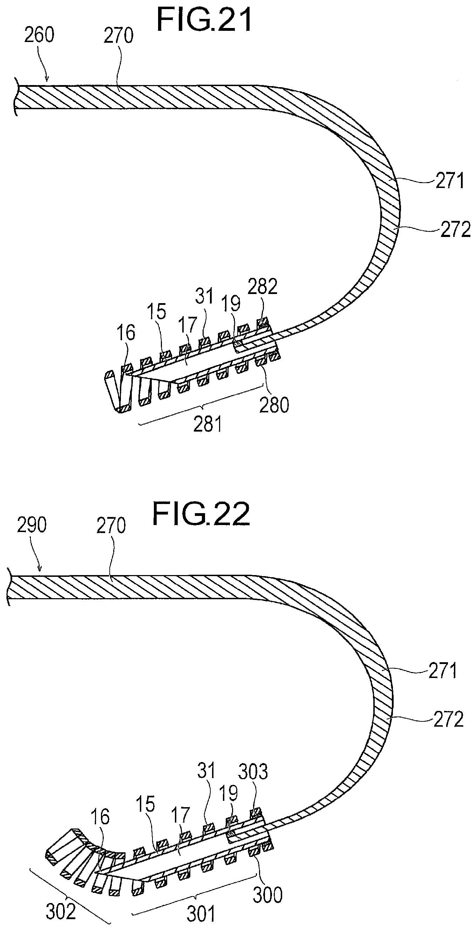

   FIG. 21 is a sectional view depicting a guide wire of a twelfth modification.
[FIG. 22]
   FIG. 22 is a sectional view depicting a guide wire of a thirteenth modification.
[FIG. 23]

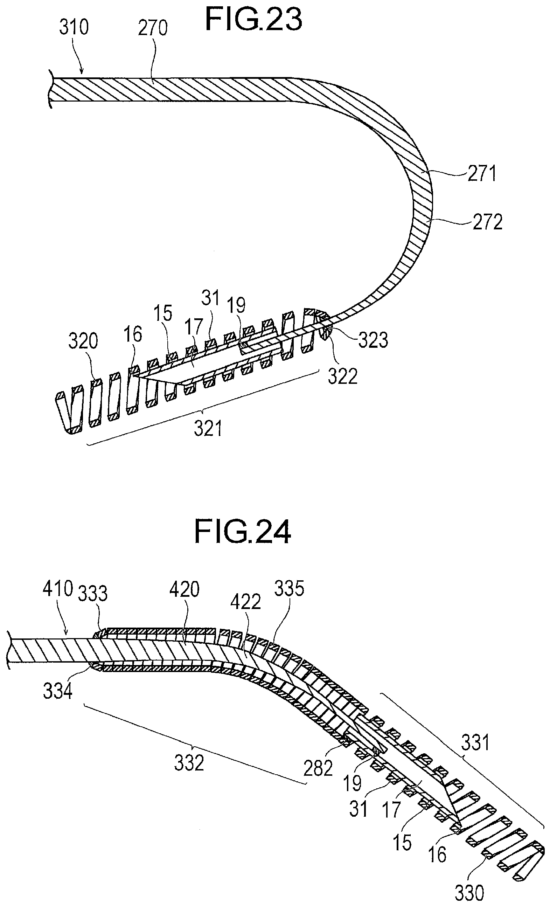

   FIG. 23 is a sectional view depicting a guide wire of a fourteenth modification.
[FIG. 24]
   FIG. 24 is a sectional view depicting a guide wire of a fifteenth modification.
[FIG. 25]

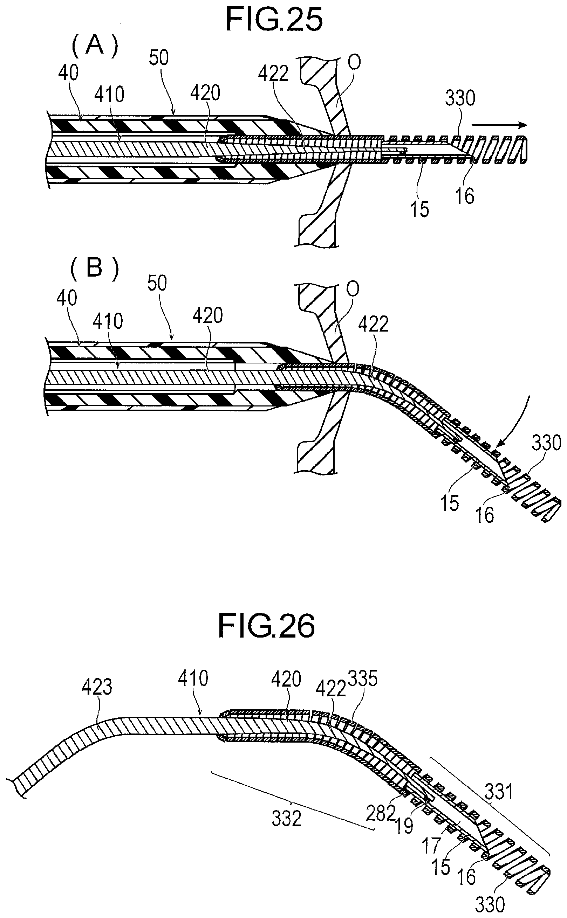

   FIG. 25 illustrates sectional views depicting a state when puncture is performed by the fifteenth modification, in which (A) depicts a state in which an oval fossa is punctured, and (B) depicts a state in which the guide wire is curved.
[FIG. 26]
   FIG. 26 is a sectional view depicting the guide wire of the fifteenth modification.
[FIG. 27]

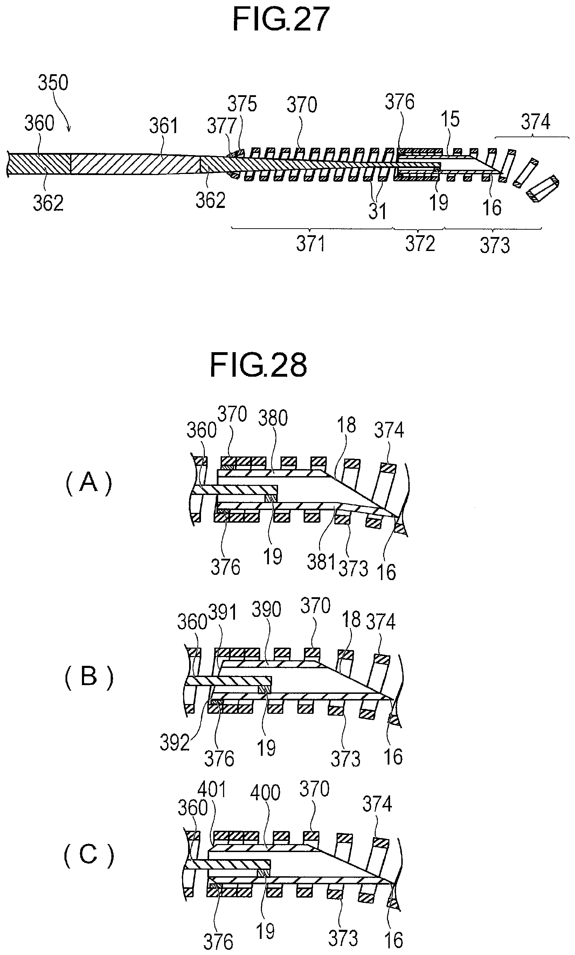

   FIG. 27 is a sectional view depicting a guide wire of a sixteenth modification.
[FIG. 28]
   FIG. 28 illustrates sectional views depicting modifications of a guide wire, in which (A) depicts a seventeenth modification, (B) depicts an eighteenth modification, and (C) depicts a nineteenth modification.
[FIG. 29]

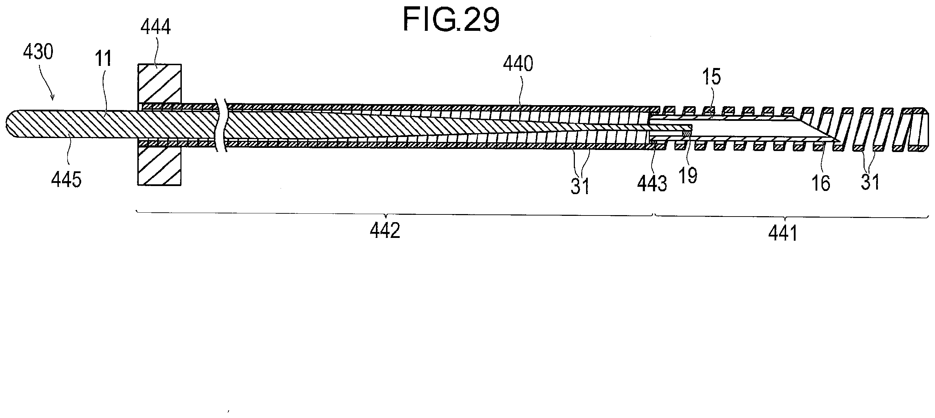

   FIG. 29 is a sectional view depicting a guide wire of a twentieth modification.
[FIG. 30]

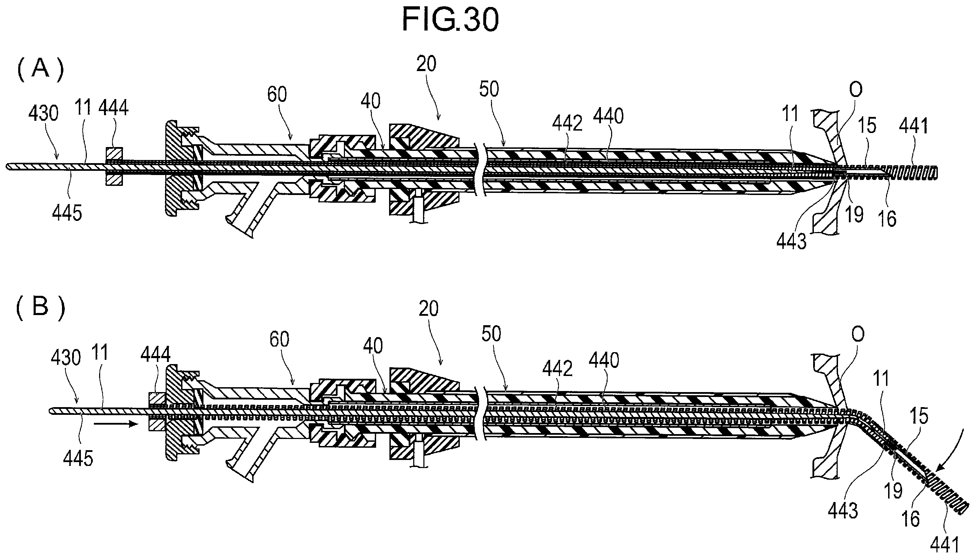

   FIG. 30 illustrates sectional views depicting a state when puncture is performed by the twentieth modification, in which (A) depicts a state in which an oval fossa is punctured, and (B) depicts a state in which the guide wire is curved.

### Mode for Carrying Out the Invention

An embodiment of the present invention will be described below referring to the drawings. Note that the dimensional ratios in the drawings may be exaggerated and may be different from the actual ones for convenience of explanation. Herein, the side of insertion of a device into a blood vessel is referred to as the "distal side" and the operator's hand side for operating the device is referred to as the "proximal side."

A medical device 1 according to the embodiment of the present invention is used for forming an oval fossa O with a hole for communication from a right atrium R to a left atrium L (see FIG. 5) by a guide wire 10 having a puncturing function. The hole may be a cut. In the case where a hole is present in the oval fossa O, an ablation catheter percutaneously inserted into a large vein can be guided to the right atrium R and then can be inserted through the hole into the left atrium L, and the periphery of an opening of a pulmonary vein can be ablated. In other words, the medical device 1 is a device for forming the oval fossa with an access route for the ablation catheter.

As depicted in FIGS. 1 to 3, the medical device 1 includes a guide wire 10 having a puncturing function, and a sheath assembly 20 in which the guide wire 10 can be inserted. The sheath assembly 20 includes an inserter 60 in which to insert the guide wire 10, a dilator 40 in which to insert the inserter 60, and an outer sheath 50 in which to insert the dilator 40.

The guide wire 10 is an elongate device for guiding a sheath assembly 20 including the dilator 40 or the ablation catheter to a desired position in a blood vessel. Further, the guide wire 10 has a function of puncturing the oval fossa O. The guide wire 10 includes an elongate shaft portion 11, a puncture portion 15 having a sharp needle portion 16, and a cover portion 30 which accommodates the puncture portion 15. The shaft portion 11 is an elongate wire member and includes a shaft proximal portion 12 located on the proximal side, a shaft distal portion 14 located on the distal side, and a shaft decreasing diameter portion 13 located between the shaft proximal portion 12 and the shaft distal portion 14. The shaft proximal portion 12 is a constant outer diameter portion located on the proximal side. The shaft decreasing diameter portion 13 is a part which extends from the shaft proximal portion 12 toward the distal side and which is decreased in outer diameter in a tapered form. The shaft decreasing diameter portion 13 with its outer diameter decreased in a tapered form has physical properties such as bending rigidity gradually varying along the axial direction. Therefore, the shaft decreasing diameter portion 13 can restrain generation of kinking or the like liable to be generated due to a sharp change in physical properties. In addition, the bending rigidity of the shaft decreasing diameter portion 13 is gradually decreased along the axial direction. Therefore, the shaft portion 11 has high pressing-in performance and delivery performance in a meandering blood vessel. The shaft distal portion 14 is a constant outer diameter portion extending from the shaft decreasing diameter portion 13 toward the distal side. The outer diameter of the shaft distal portion 14 is smaller than the outer diameter of the shaft proximal portion 12. Note that the outer diameter of the shaft distal portion 14 may not be constant.

The constituent material of the shaft portion 11 is preferably flexible but still rigid to a certain extent. Examples of materials which can be preferably used include metals such as stainless steel, tantalum, titanium, platinum, gold, and tungsten, shape memory alloys to which a shape memory effect or superelasticity is imparted by a heat treatment, polyolefins such as polyethylene and polypropylene, polyamides, polyesters such as polyethylene terephthalate, fluoro-polymers such as PTFE (polytetrafluoroethylene) and ETFE (ethylene-tetrafluoroethylene copolymer), PEEK (polyether ether ketone), and polyimides. As the shape memory alloys, those based on Ni-Ti, Cu-Al-Ni, Cu-Zn-Al, or the like can be preferably used. In addition, the shaft portion 11 may include an X-ray contrast material. Preferable examples of the X-ray contrast material include at least one metal or two or more alloys selected from the group consisting of gold, platinum, iridium, tungsten, their alloys, and silver-palladium alloys.

The axial length of the shaft portion 11 is, for example, 300 to 5,000 mm, preferably 1,000 to 3,000 mm, and more preferably 1,500 to 2,500 mm. The outer diameter of the shaft distal portion 14 is, for example, 0.04 to 0.8 mm, preferably 0.08 to 0.4 mm, and more preferably 0.12 to 0.2 mm. The outer diameter of the shaft proximal portion 12 is, for example, 0.3 to 1.0 mm, preferably 0.4 to 0.8 mm, and more preferably 0.5 to 0.6 mm.

The puncture portion 15 is a circular pipe having a sharp needle portion 16 for puncturing living body tissue. The puncture portion 15 is provided with a through-hole 17 penetrating from the proximal side to the distal side. The puncture portion 15 is fixed to a distal portion of the shaft portion 11. The puncture portion 15 is provided at the distal end thereof with an inclined surface 18 inclined relative to the center axis. The inclined surface 18 is the distal end of the sharp needle portion 16 for puncturing living body tissue. The distal end of the shaft distal portion 14 is disposed inside the puncture portion 15. The distal end of the shaft distal portion 14 is fixed to an inner peripheral surface of the puncture portion 15 by welding, adhesion, or the like with a needle fixing portion 19. The inner diameter of the puncture portion 15 is larger than the outer diameter of the shaft distal portion 14. The needle fixing portion 19 is provided only at a part in the circumferential direction of the shaft distal portion 14. Therefore, the shaft distal portion 14 and the needle fixing portion 19 do not hinder flowing property of a fluid inside the puncture portion 15. Note that a plurality of the needle fixing portions 19 may be provided. In addition, the axis of the puncture portion 15 and the axis of the shaft distal portion 14 preferably coincide substantially with each other. The axis of the puncture portion 15 and the axis of the shaft distal portion 14 may not coincide with each other. The shape of the needle portion 16 of the puncture portion 15 is not particularly limited, insofar as the needle portion 16 can puncture living body tissue. For example, a conical shape, a knife-type flat plate, or a shovel-shaped curved plate may be adopted. Therefore, the puncture portion 15 may not be formed with the through-hole 17. Besides, the sectional shape of the puncture portion 15 may not be circular. In addition, the puncture portion 15 may be integral with the shaft portion 11 in structure. The puncture portion 15 may be an energy part such as an electrode capable of releasing energy.

The axial length of the puncture portion 15 is preferably in such an extent as not to hamper flexibility of the guide wire 10 in a blood vessel. The axial length of the puncture portion 15 is, for example, 2 to 10 mm, preferably 3 to 8 mm, and more preferably 4 to 6 mm. The outer diameter of the puncture portion 15 is, for example, 0.3 to 1.0 mm, preferably 0.4 to 0.8 mm, and more preferably 0.5 to 0.6 mm. The inner diameter of the puncture portion 15 is, for example, 0.1 to 0.9 mm, preferably 0.2 to 0.7 mm, and more preferably 0.3 to 0.5 mm. An inclination angle α1 of the inclined surface 18 of the puncture portion 15 relative to the center axis can be, for example, 3 to 45 degrees, preferably 5 to 40 degrees, and more preferably 10 to 35 degrees.

The constituent material of the puncture portion 15 is preferably rigid to a certain extent. Examples of preferably usable materials include metals such as stainless steel, tantalum, titanium, platinum, gold, and tungsten, polyolefins such as polyethylene and polypropylene, polyamides, polyesters such as polyethylene terephthalate, fluoro-polymers such as PTFE (polytetrafluoroethylene) and ETFE (ethylene-tetrafluoroethylene copolymer), PEEK (polyether ether ketone), and polyimides. In addition, the puncture portion 15 may include an X-ray contrast material. Preferred examples of the X-ray contrast material include at least one metal or two or more alloys selected from the group consisting of gold, platinum, iridium, tungsten, their alloys, and silver-palladium alloys. The surface of the puncture portion 15 may be subjected to silicone coating or hydrophilic coating.

The cover portion 30 is a generally tubular elastically deformable member that accommodates the puncture portion 15 in a protrudable manner. The cover portion 30 is formed of a wire member 31 in a helical shape. An end portion on the distal side of the wire member 31 is subjected to brazing to fill up a gap between adjacent loops of the wire member 31 for restraining damage to living body tissue contacted thereby. Alternatively, at the end portion on the distal side of the wire member 31, adjacent loops of the wire member 31 are brought into contact with each other and subjected to welding, after which an outer edge portion is subjected to curved surface processing to be formed smoothly. The cover portion 30 includes a coarse pitch portion 33 in which a helix pitch distance is long, a dense pitch portion 34 in which the helix pitch distance is shorter than that of the coarse pitch portion 33, and a curved portion 35 located on the distal side relative to the dense pitch portion 34. The pitch distance means a moving distance in the axial direction when a helix is wound by 360 degrees in the circumferential direction. The coarse pitch portion 33, the dense pitch portion 34, and the curved portion 35 are formed from a single continuous wire member 31. Note that the coarse pitch portion 33, the dense pitch portion 34, and the curved portion 35 may be formed from a plurality of wire members. In addition, the wire member 31 may not be provided with the dense pitch portion 34 in a helical shape with no gap. Besides, the cover portion 30 may be formed of an elastically deformable resin tube or a meshed tube that is obtained by knitting wire members. The meshed tube may have at least part thereof embedded or not embedded in a resin tube.

The coarse pitch portion 33 is formed of the wire member 31 in a helical shape with a gap between adjacent loops thereof. A proximal end 32 of the coarse pitch portion 33 is fixed to the shaft portion 11 by a joint part 36 including a solder, an adhesive, a melted material obtained by welding, or the like. The joint part 36 is filling up a step and a gap between the proximal end 32 and the shaft portion 11. The coarse pitch portion 33 has a substantially constant outer diameter and inner diameter along the axial direction. The coarse pitch portion 33 surrounds a proximal portion of the shaft proximal portion 12, the shaft decreasing diameter portion 13, and a proximal portion of the shaft distal portion 14. The axis of the helix of the coarse pitch portion 33 coincides substantially with axes of the shaft proximal portion 12, the shaft decreasing diameter portion 13, and the shaft distal portion 14. The inner diameter of the coarse pitch portion 33 is larger than the outer diameter of the shaft portion 11 located inside the coarse pitch portion 33. Therefore, a flow path for a fluid to flow therethrough is formed between the shaft portion 11 and the coarse pitch portion 33. As illustrated in FIG. 7(A), the coarse pitch portion 33 can be shrunk in the axial direction such that the gap between adjacent loops of the wire member 31 aligned in the axial direction is decreased. When the coarse pitch portion 33 is shrunk in the axial direction, the dense pitch portion 34 and the curved portion 35 located on the distal side relative to the coarse pitch portion 33 are moved toward the proximal side relative to the puncture portion 15. As a result, the needle portion 16 of the puncture portion 15 accommodated inside the dense pitch portion 34 is protruded to the distal side through the curved portion 35.

As illustrated in FIG. 3, the dense pitch portion 34 is formed of the wire member 31 in a helical shape with no gap between adjacent loops thereof. The dense pitch portion 34 has a substantially constant outer diameter and inner diameter along the axial direction. The dense pitch portion 34 surrounds the puncture portion 15 and a part of the shaft distal portion 14 located inside the puncture portion 15. The axis of the helix of the dense pitch portion 34 coincides substantially with axes of the puncture portion 15 and the shaft distal portion 14. Note that the dense pitch portion 34 may have a gap between adjacent loops of the wire member 31 aligned in the axial direction, insofar as a pitch distance of the dense pitch portion 34 is shorter than that of the coarse pitch portion 33. In addition, the pitch distances of the dense pitch portion 34 and the coarse pitch portion 33 may coincide with each other. Besides, the pitch distance of the dense pitch portion 34 may be longer than the pitch distance of the coarse pitch portion 33. The inner diameter of the dense pitch portion 34 is slightly larger than the outer diameter of the puncture portion 15. This ensures that axial deformation of the dense pitch portion 34 is not hindered by a frictional force with the puncture portion 15. The inner peripheral surface of the dense pitch portion 34 may be in contact or may not be in contact with the outer peripheral surface of the puncture portion 15.

The curved portion 35 is formed of the wire member 31 in a helical shape such as to have a curved tubular shape as a whole. The curved portion 35 is located from the vicinity of the distal end of the needle portion 16 to a distal-side end surface of the cover portion 30. The curved portion 35 may be a part between the vicinity of the distal end of the needle portion 16 and the distal-side end surface of the cover portion 30. In that case, the curved portion 35 is located between rectilinear portions of the cover portion 30. The angle β by which the cover portion 30 is curved in the curved portion 35 is not particularly limited but is preferably equal to or more than 45 degrees and equal to or less than 180 degrees, more preferably equal to or more than 90 degrees and equal to or less than 180 degrees. When the angle β is equal to or more than 90 degrees, at the time of pressing the guide wire 10 into a living body, the distal end of the guide wire 10 is not liable to collide against the living body tissue, so that damage to the living body tissue can be restrained. When the angle β is equal to or less than 180 degrees, it is easy to direct the guide wire 10 in a living body into a desired direction by using the curvature of the curved portion 35, so that operability is enhanced. A distal-side end surface of the curved portion 35, that is, a distal-side end surface of the cover portion 30, is reduced in diameter in a tapered form toward the distal side. Since the curved portion 35 is curved, wideness of the gap between adjacent loops of the wire member 31 is different on the inside and the outside of the curvature. Adjacent loops of the wire member 31 of the curved portion 35 are arranged with no gap therebetween on the inside of the curvature. Note that the adjacent loops of the wire member 31 may be arranged with a gap therebetween on the inside of the curvature. The adjacent loops of the wire member 31 of the curved portion 35 are arranged with a wider gap therebetween on the outside of the curvature than on the inside of the curvature. Note that the wire member 31 of the curved portion 35 may be formed into a helical shape with no gap between adjacent loops thereof on both the inside and the outside of the curvature in the curved state. In this case, the wire member 31 differs in shape depending on the position.

The direction in which the curved portion 35 is curved coincides with direction in which the sharp needle portion 16 is located at the inclined surface 18. Therefore, when the needle portion 16 is moved in the curved portion 35 toward the distal side, the needle portion 16 can be restrained from contacting the inner peripheral surface of the curved portion 35. Consequently, the needle portion 16 can be restrained from unintentionally protruding from the curved portion 35 to damage the living body tissue, so that safety is enhanced.

The curved portion 35 is curved in a natural state in which an external force from gravity, blood flow, or the like is not acting thereon. Note that the curved portion 35 may not be curved in the natural state, insofar as it is bent in the use environment. For example, the curved portion 35 may be curved due to its own weight acting thereon or may be curved due to a force from the blood flow acting thereon. Since the cover portion 30 is flexible, even if the curved portion 35 is not curved in the natural state, the curved portion 35 may be formed by action thereon of an external force from its own weight, blood flow, or the like.

The sectional shape of the wire member 31 constituting the cover portion 30, in a section orthogonal to the extending direction of the wire member 31, is a rectangle (or a square). Therefore, when the cover portion 30 is shrunk in the axial direction, adjacent loops of the wire member 31 aligned in the axial direction contact one another in wide areas. For this reason, the cover portion 30 shrunk in the axial direction is high in capability of transmitting forces in the axial direction. Note that the sectional shape of the wire member 31 constituting the cover portion 30 may not be a rectangle or a square. For example, the sectional shape may be a circle, an ellipse, a parallelogram, a trapezoid, or the like.

The guide wire 10 includes a guide wire proximal portion 37, a guide wire intermediate part 38, and a guide wire distal portion 39 aligned in this order from the proximal side in the axial direction. The guide wire proximal portion 37 includes the shaft proximal portion 12 having the constant outer diameter, and a proximal portion of the cover portion 30. The guide wire intermediate part 38 includes the shaft portion 11 and the cover portion 30 which are located on the distal side relative to the proximal end of the shaft decreasing diameter portion 13 and located on the proximal side relative to the proximal end of the puncture portion 15. The guide wire distal portion 39 includes the puncture portion 15, the cover portion 30, and the shaft portion 11 which are located on the distal side relative to the proximal end of the puncture portion 15. A part of the guide wire distal portion 39 is higher in bending rigidity than the guide wire intermediate part 38. The guide wire proximal portion 37 is higher in bending rigidity than a part of the guide wire intermediate part 38. The length of the guide wire 10 can be, for example, 300 to 5,000 mm.

The cover portion 30 is formed by winding the rectilinear wire member 31 into a helical form. In addition, the cover portion 30 may be cut out from a circular pipe by laser processing or the like.

The constituent material of the cover portion 30 is preferably elastically deformable but still rigid to a certain extent. Preferably usable materials include shape memory alloys to which a shape memory effect or superelasticity is imparted by a heat treatment, metals such as stainless steel, tantalum, titanium, platinum, gold, and tungsten, polyolefins such as polyethylene and polypropylene, polyamides, polyesters such as polyethylene terephthalate, fluoro-polymers such as PTFE (polytetrafluoroethylene) and ETFE (ethylene-tetrafluoroethylene copolymer), PEEK (polyether ether ketone), and polyimides. As the shape memory alloys, those based on Ni-Ti, Cu-Al-Ni, Cu-Zn-Al, or the like can be preferably used. In addition, the cover portion 30 may include an X-ray contrast material. Preferred examples of the X-ray contrast material include at least one metal or two or more alloys selected from the group consisting of gold, platinum, iridium, tungsten, their alloys, and silver-palladium alloys. Besides, since the cover portion 30 is formed into a helical shape, its ruggedness is increased, whereby it can have high ultrasonic imaging properties. The cover portion 30 may be plastically deformed. For example, if at least the curved portion 35 of the cover portion 30 is plastically deformable, the curvature of the curved portion 35 can be adjusted by the operator according to the purpose before procedure.

In general, the guide wire 10 has high flexibility while having a certain degree of rigidity such that the guide wire 10 can be pressed forward in a meandering body lumen without damaging the body lumen in which it is inserted. Therefore, when a proximally directed force is received at the distal end of the cover portion 30 in a state in which flexure (deformation in the radial direction) of the guide wire 10 in the present embodiment is not restricted, some part of the guide wire 10 is flexed, and the force is relieved from a distal portion of the cover portion 30 to other parts. Further, since the distal portion of the guide wire 10 is curved, a force is not easily exerted on the distal end, and a force for compressing the cover portion 30 in the axial direction is not liable to act thereon. For this reason, even when a proximally directed force is received at the distal end of the cover portion 30, a force for compressing the cover portion 30 in the axial direction does not act on the cover portion 30. Therefore, the cover portion 30 is flexed by receiving the proximally directed force in a state in which flexure is free, and maintains a state of accommodating the needle portion 16.

As depicted in FIGS. 1 and 2, the inserter 60 permits the guide wire 10 to be inserted therein and can be inserted in the dilator 40. In order to suitably puncture the oval fossa O, the medical device 1 should be provided with an appropriate angle and rigidity such as to suitably face the oval fossa O in the right atrium R. The inserter 60, by being inserted in the dilator 40, can enhance the rigidity and angle of the medical device 1. For example, a known puncture needle is provided, on the proximal side of the sharp needle portion, with a metallic shaft larger in outer diameter than the needle portion and bent at a predetermined angle. In the present embodiment, the inserter 60 is separately provided for imparting appropriate angle and rigidity to the medical device 1. Since the inserter 60 is independent from the guide wire 10, it is possible, after puncture of the oval fossa O, to withdraw only the inserter 60, leaving the guide wire 10. Therefore, transport of the outer sheath 50 through the guide wire 10 can be performed more smoothly. In addition, with the inserter 60 abutted on an inner diameter decreasing part 45 of the dilator 40 and disposed at a dilator proximal portion 44, spaces inside the inserter 60 and a dilator distal portion 43 are continuous with each other with a substantially uniform inner diameter. Therefore, the insertability of the guide wire 10 in spaces inside the inserter 60 and the dilator 40 is enhanced. Consequently, the guide wire 10 can be restrained from being caught in the inside of the dilator 40 having the inner diameter decreasing part 45 for inserting a known puncture needle.

The inserter 60 includes an inserter main body 61 in which the guide wire 10 can be inserted, and an inserter hub 62 provided on the proximal side of the inserter main body 61.

The inserter main body 61 is a tube body, in which the guide wire 10 can be inserted. An outer surface of the inserter main body 61 may be coated with a low friction material such that the inserter main body 61 can make contact with the dilator 40 with low friction. Examples of the low friction material include fluoro-polymers such as PTFE (polytetrafluoroethylene) and ETFE (ethylene-tetrafluoroethylene copolymer) and silicone oils. In addition, an inner surface of the inserter main body 61 may be coated with a low friction material such that the inserter main body 61 can make contact with the guide wire 10 with low friction.

The inserter main body 61 is provided at a distal portion thereof with an inserter bent portion 63 bent at a predetermined angle in a natural state. An angle β1 of the inserter bent portion 63 relative to the proximal portion of the inserter main body 61 is not particularly limited and is, for example, 20 to 90 degrees, preferably 30 to 85 degrees, and more preferably 40 to 80 degrees. The inserter bent portion 63 functions to direct the needle portion 16 of the guide wire 10 inserted in the right atrium R toward the oval fossa O. An edge part at an outer periphery of a distal-side end portion of the inserter main body 61 is preferably processed into a curved surface such that the inserter main body 61 can be smoothly inserted into the dilator 40.

Examples of the constituent material of the inserter main body 61 include shape memory alloys to which a shape memory effect or superelasticity is imparted by a heat treatment, metals such as stainless steel, tantalum, titanium, platinum, gold, and tungsten, polyolefins such as polyethylene and polypropylene, polyamides, polyesters such as polyethylene terephthalate, fluoro-polymers such as PTFE (polytetrafluoroethylene) and ETFE (ethylene-tetrafluoroethylene copolymer), PEEK (polyether ether ketone), and polyimides. In addition, the inserter main body 61 may include an X-ray contrast material.

The inserter hub 62 includes a tube part 64 connected to a proximal portion of the inserter main body 61, a first connection part 65 connected to the dilator 40, a port portion 66, a valve body 67, and a rotational fixing portion 68. The tube part 64 is located at a distal-side end portion of the inserter hub 62. The tube part 64 is capable of communicating with a space inside the inserter main body 61 connected therewith. The first connection part 65 is a tubular member rotatably provided at an outer peripheral surface of the tube part 64. An inner peripheral surface of the first connection part 65 is formed with a female connector capable of screw engagement with a second connection part 41 provided at a proximal portion of the dilator 40. The port portion 66 permits, for example, a three-way cock, a syringe, or the like to be connected thereto. The port portion 66 permits priming of the space inside the inserter main body 61 and permits a contrast medium, a chemical, or the like to be injected into the inserter main body 61. In addition, by using the port portion 66, blood in the living body can be led to the exterior and blood pressure can be measured. By measurement of blood pressure, it can be accurately confirmed that the needle portion 16 has reached a desired position. The direction in which the port portion 66 is provided relative to the inserter hub 62 coincides with the direction in which the inserter bent portion 63 is bent. Therefore, by confirming the position of the port portion 66 of the inserter hub 62, the direction in which the inserter bent portion 63 is bent can be easily grasped even outside the body. The valve body 67 is located inside a proximal portion of the inserter hub 62. The guide wire 10 slidably penetrates the valve body 67. The valve body 67 makes slidable contact with an outer peripheral surface of the guide wire 10. The valve body 67 is, for example, a member in which a cut is formed in the center of a disk-shaped elastic body. The elastic body may be, for example, a natural rubber, a silicone rubber, or any of various elastomers. The valve body 67 restrains blood from leaking from the inserter main body 61 and restrains air from entering the body. The rotational fixing portion 68 screw engages with an outer peripheral surface of the inserter hub 62. The rotational fixing portion 68 can be moved in the axial direction by rotating relative to the outer peripheral surface of the inserter hub 62. The rotational fixing portion 68 has a presser portion 69 that protrudes to the distal side to make contact with the valve body 67. When the rotational fixing portion 68 rotates and moves toward the distal side, the presser portion 69 presses the valve body 67 to compress the valve body 67. As a result, the valve body 67 can fix, in an unmovable manner, the guide wire 10 that penetrates the valve body 67. In addition, when the rotational fixing portion 68 rotates and moves toward the proximal side, the presser portion 69 moves in a direction for spacing away from the valve body 67. As a result, compression of the valve body 67 by the presser portion 69 is released, and the guide wire 10 becomes able to move the valve body 67 slidably. The inserter 60 with the guide wire 10 inserted therein permits a contrast medium, a chemical, or the like to be injected via the port portion 66 and permits blood pressure to be measured. Therefore, the inserter 60 is high in handiness.

The constituent material of the inserter hub 62 is not particularly limited. Examples of the material include rigid resins such as polycarbonate, polyethylene, polypropylene, and ABS resins (generic name of acrylonitrile-butadiene-styrene copolymer synthetic resins), and metals such as stainless steel.

The dilator 40 is used for expanding a hole formed in the oval fossa O by the guide wire 10. The dilator 40 is provided at a distal-side end portion thereof with a tapered portion 42 decreased in diameter in a tapered form toward the distal side. The space inside the dilator 40 is opening at an end portion where the diameter of the tapered portion 42 is the most decreased. An inclination angle α2 of the tapered portion 42 relative to the center axis can be, for example, 1 to 20 degrees, preferably 3 to 15 degrees, and more preferably 4 to 10 degrees. The dilator 40 is provided at an outer peripheral surface of a proximal portion thereof with the second connection part 41 which can be connected to the first connection part 65 of the inserter hub 62. The second connection part 41 is a male connector.

The dilator 40 includes a dilator distal portion 43 located on the distal side and small in inner diameter, and a dilator proximal portion 44 located on the proximal side and larger in inner diameter than the dilator distal portion 43. Between the dilator distal portion 43 and the dilator proximal portion 44, there is provided an inner diameter decreasing part 45 decreasing in inner diameter toward the distal side. An inner peripheral surface of the dilator proximal portion 44 is capable of making slidable close contact with an outer peripheral surface of the inserter main body 61. When the inserter main body 61 is inserted in the dilator proximal portion 44, the inserter main body 61 is fixed to an accurate position relative to the dilator 40. In this instance, a distal-side end portion of the inserter main body 61 makes contact with the inner diameter decreasing part 45. Note that the inner diameter of the dilator 40 may be constant along the axial direction.

The dilator 40 is provided, at a distal portion thereof, with a dilator bent portion 46 bent at a predetermined angle in a natural state. An angle β2 of the dilator bent portion 46 relative to a proximal portion of the dilator 40 is not particularly limited and is, for example, 10 to 70 degrees, preferably 20 to 60 degrees, and more preferably 30 to 50 degrees. The dilator bent portion 46 functions to direct the puncture portion 15 of the guide wire 10 inserted in the right atrium R and the tapered portion 42 of the dilator 40 toward the oval fossa O.

The length of the dilator 40 can be, for example, 150 to 1,500 mm. The outer diameter of the dilator 40 can be, for example, 2 to 6 mm. The inner diameter of the dilator distal portion 43 can be, for example, 0.5 to 1.5 mm. The inner diameter of the dilator proximal portion 44 can be, for example, 1.0 to 2.0 mm. The length of the dilator distal portion 43 can be, for example, 1 to 15 mm, preferably 2 to 12 mm, and more preferably 3 to 10 mm. A radial clearance between an inner peripheral surface of the dilator proximal portion 44 and an outer peripheral surface of the inserter main body 61 can be, for example, 0.03 to 0.1 mm.

The constituent material of the dilator 40 is preferably flexible. Examples of the material include polyolefins such as polyethylene and polypropylene, polyamides, polyesters such as polyethylene terephthalate, fluoro-polymers such as PTFE (polytetrafluoroethylene) and ETFE (ethylene-tetrafluoroethylene copolymer), PEEK (polyether ether ketone), polyimides, shape memory alloys, and metals such as stainless steel, tantalum, titanium, platinum, gold, and tungsten. In addition, the dilator 40 may include an X-ray contrast material or an ultrasonic contrast material.

The outer sheath 50 provides an access route for the ablation catheter. The outer sheath 50 includes a sheath main body 51, a hub 54 connected to a proximal portion of the sheath main body 51, a sheath port portion 56 connected to the hub 54, and a valve body 55 inside the hub 54.

The sheath main body 51 is an elongate pipe body that accommodates the dilator 40 in an axially movable manner. The sheath main body 51 has an inner peripheral surface making smooth slidable contact with the dilator 40. The sheath main body 51 is provided, at a distal portion thereof, with a sheath bent portion 52 bent at a predetermined angle in a natural state. An angle β3 of the sheath bent portion 52 relative to a proximal portion of the sheath main body 51 is not particularly limited and is, for example, 10 to 180 degrees, preferably 30 to 150 degrees, and more preferably 45 to 135 degrees. The sheath bent portion 52 functions to direct the puncture portion 15 of the guide wire 10 inserted in the right atrium R and a distal portion of the sheath main body 51 toward the oval fossa O.

The sheath main body 51 is provided, at a distal-side end portion thereof, with a sheath tapered portion 53 decreased in diameter in a tapered form toward the distal side. A space inside the sheath main body 51 is opening at the end portion where the diameter of the sheath tapered portion 53 is the most decreased. An inclination angle α3 of the sheath tapered portion 53 relative to the center axis can be, for example, 1 to 15 degrees, preferably 2 to 10 degrees, and more preferably 3 to 7 degrees. In the sheath assembly 20 having the dilator 40 inserted in the outer sheath 50, the sheath tapered portion 53 is located on the proximal side of the tapered portion 42 of the dilator 40 and can be located such as to be continuous with the tapered portion 42. An inner peripheral surface of the sheath main body 51 preferably has a clearance between itself and an outer peripheral surface of the dilator 40 such that the outer peripheral surface of the dilator 40 makes slidable contact therewith.

The sheath main body 51 permits the dilator 40 to penetrate it over the whole length thereof. Therefore, the axial length of the sheath main body 51 is shorter than that of the dilator 40.

The length of the sheath main body 51 can be, for example, 400 to 1,000 mm. The outer diameter of the sheath main body 51 can be, for example, 2.5 to 7.0 mm. The inner diameter of the sheath main body 51 can be, for example, 2 to 6 mm. A radial clearance between the inner peripheral surface of the sheath main body 51 and the outer peripheral surface of the dilator 40 can be, for example, 0.1 to 0.5 mm.

The constituent material of the sheath main body 51 is preferably a flexible material. Examples of the material include polyolefins such as polyethylene and polypropylene, polyamides, polyesters such as polyethylene terephthalate, fluoro-polymers such as PTFE (polytetrafluoroethylene) and ETFE (ethylene-tetrafluoroethylene copolymer), PEEK (polyether ether ketone), and polyimides. In addition, the constituent material of the sheath main body 51 may include an X-ray contrast material, a metallic braid, a coil, or the like.

The hub 54 is provided at a proximal portion of the sheath main body 51, and communicates with the space inside the sheath main body 51. The dilator 40 penetrates the hub 54. The sheath port portion 56 is connected to the hub 54 and communicates with the space inside the sheath main body 51 via a space inside the hub 54. The sheath port portion 56 has a three-way cock 57 at an end portion thereof. With a syringe or the like connected to the three-way cock 57, it is possible to perform priming of the space inside the sheath main body 51 or to inject a contrast medium, a chemical, or the like into the sheath main body 51.

The valve body 55 is a member for sealing the spaces inside the hub 54 and the sheath main body 51. The valve body 55 is flexibly deformable and is disposed on the inner peripheral surface of the hub 54. The valve body 55 makes slidable contact with the outer peripheral surface of the dilator 40. In addition, the valve body 55, in a state in which the dilator 40 is inserted therein, can press the dilator 40 by an elastic force and can fix the dilator 40 and the outer sheath 50. Note that, even when fixed by the valve body 55, if the dilator 40 and the outer sheath 50 are grasped and forces are exerted thereon, they can be relatively moved in the axial direction. With the dilator 40 withdrawn from the hub 54, a hole part of the valve body 55 in which the dilator 40 has been inserted is closed, and the space inside the hub 54 is sealed by the valve body 55 from the proximal side. The valve body 55 is, for example, a member in which a cut is formed in the center of a disk-shaped elastic body. The elastic body may be, for example, a natural rubber, a silicone rubber, or any of various elastomers. The valve body 55 restrain blood from leaking through the outer sheath 50 and restrains air from entering the living body while permitting the dilator 40 to be inserted and withdrawn.

In a state in which the inserter 60, the dilator 40, and the outer sheath 50 are combined together, it is preferable that the positions, bending directions, and bending angles of the inserter bent portion 63, the dilator bent portion 46, and the sheath bent portion 52 coincide or substantially coincide with one another. As a result, the needle portion 16 of the guide wire 10 can be projected in a desired direction.

A method of opening a hole in the oval fossa O, which is not part of the invention, by use of the medical device 1 according to the present embodiment to thereby provide an access route for the ablation catheter will be described below referring to a flow chart of FIG. 4.

First, a needle is made to puncture a femoral vein, and a general-purpose guide wire (not illustrated) is inserted in the needle. Next, the needle is withdrawn, and an assembly having the dilator 40 inserted inside the outer sheath 50 is inserted into the blood vessel along the general-purpose guide wire (step S10). The assembly is pressed forward while causing the general purpose guide wire to precede, and, after the assembly comes beyond the right atrium R and the distal end of the dilator 40 has reached a superior vena cava, the general-purpose guide wire is withdrawn. Note that the guide wire 10 may be used in place of the general-purpose guide wire.

Next, the inserter 60 and the guide wire 10 are inserted into the assembly including the outer sheath 50 and the dilator 40 (step S11) (see FIG. 6(A)). In this instance, the distal end of the guide wire 10 is located between a distal-side opening of the dilator 40 and a distal-side opening of the inserter 60. The first connection part 65 of the inserter 60 is connected to the second connection part 41 of the dilator 40. Note that the inserter 60 may not be connected to the dilator 40. Therefore, the first connection part 65 and the second connection part 41 may not be provided. The guide wire 10 has such a structure that the outer peripheral surface of the cover portion 30 accommodating the puncture portion 15 is not completely bound. Therefore, when the guide wire 10 is moved inside the inserter 60 and the dilator 40, the puncture portion 15 of the guide wire 10 is maintained in the state of being accommodated in the cover portion 30. Next, the sheath assembly 20 is retracted and drawn into the right atrium R. As a result, as depicted in FIG. 5 and FIG. 6(A), the distal-side end portion of the sheath assembly 20 is automatically led into the vicinity of the oval fossa O (step S12).

Subsequently, while observing the inside of the left atrium L and the right atrium R by an ICE (Intra cardiac echo catheter), the sheath assembly 20 is pressed toward the distal side as depicted in FIG. 6(B) (step S13). As a result, the oval fossa O is pressed toward the left atrium L side by the dilator 40 and protrudes to the left atrium L side. In this instance, since the distal portions of the outer sheath 50, the dilator 40, and the inserter 60 are bent, a distal-side end portion of the dilator 40 can be easily directed toward the oval fossa O. Note that the oval fossa O may not be put into the state of protruding to the left atrium L side. The sheath assembly 20 is reinforced by the inserter 60 inserted in the dilator 40. For this reason, the sheath assembly 20 can be directed toward the oval fossa O with such rigidity and at such an angle as to be suitable for puncture even without insertion of a known bent puncture needle high in rigidity.

Next, as depicted in FIG. 7(A), a proximal portion of the guide wire 10 located outside of the body is pressed into the inserter hub 62. The curved portion 35 of the cover portion 30 is stretched rectilinearly in the inside of the inserter 60. The wire member 31 of the curved portion 35 thus stretched rectilinearly is formed into a helical shape with no gap between adjacent loops thereof. Note that the wire member 31 of the curved portion 35 stretched rectilinearly may be formed into a helical shape with a gap between adjacent loops thereof. When the guide wire 10 is moved inside the inserter 60 and the dilator 40 toward the distal side, the cover portion 30 located at the distal end of the guide wire 10 comes into contact with the oval fossa O (step S14). As a result, a proximally directed force acts on the distal end of the cover portion 30 located at the most distal position of the guide wire 10. In the case where the distal-side end portion of the cover portion 30 makes contact with the oval fossa O in the same plane, the needle portion 16 becomes perpendicular to the oval fossa O. As a result, the cover portion 30 is easily shrunk, the needle portion 16 is easily exposed from the cover portion 30, and the needle portion 16 easily punctures the oval fossa O. For example, in the case where the cover portion 30 is a cylinder, the whole circumference of the distal-side end portion is liable to puncture the oval fossa O upon contact with the oval fossa O. The coarse pitch portion 33 of the cover portion 30, by receiving a proximally directed force on the distal end thereof, elastically shrinks in the axial direction such as to reduce the gap between adjacent loops of the wire member 31. In the case where the gap is present between adjacent loops of the wire member 31 of the curved portion 35 stretched rectilinearly, the curved portion 35 elastically shrinks in the axial direction such as to reduce the gap between adjacent loops of the wire member 31. The cover portion 30 is surrounding the shaft decreasing diameter portion 13 decreased in outer diameter and low in rigidity and the shaft distal portion 14 on the distal side relative to the shaft decreasing diameter portion 13. Therefore, the cover portion 30 compensates for the periphery of the shaft portion 11, which is liable to flex due to a lowering in rigidity, and reduces the gaps. For this reason, puncture of the oval fossa O by the puncture portion 15 fixed to the shaft portion 11 can be performed effectively. In addition, the dense pitch portion 34 cannot shrink in the axial direction and, therefore, by being accommodated in the inside of the dilator 40 or the inserter 60, transmits axial forces effectively to permit effective puncture. Besides, after the tip of the cover portion 30 makes contact with the living body tissue and before the wire member 31 of the cover portion 30 shrinks completely, the puncture portion 15 may be exposed from the dilator 40 (elongate body) to puncture the living body tissue. In other words, a shrinking distance of the wire member 31 of the cover portion 30 is longer than the distance between the tip of the cover portion 30 and the tip of the puncture portion 15. In addition, the cover portion 30 is preferably shaped in such a manner that mere contact of only the cover portion 30 with the living body tissue cannot produce a force sufficient for puncturing the thickness, rigidity, or the like of the living body tissue and that the puncture portion 15 is liable to enter the hole formed in the living body tissue.

When the coarse pitch portion 33 shrinks in the axial direction, the dense pitch portion 34 and the curved portion 35 located on the distal side relative to the coarse pitch portion 33 are moved toward the proximal side relative to the puncture portion 15. As a result, the needle portion 16 of the puncture portion 15 accommodated in the inside of the dense pitch portion 34 is exposed from the cover portion 30 toward the distal side via the curved portion 35. Therefore, when the guide wire 10 is pressed toward the distal side, a hole can be formed in the oval fossa O by the puncture portion 15 exposed from the cover portion 30 (step S15). It is desirable that the force for shrinking the cover portion 30 in the axial direction until the needle portion 16 is exposed is greater than the frictional resistance acting on the guide wire 10 when the guide wire 10 is inserted into the dilator 40 or the living body. This ensures that when the guide wire 10 is moved in the dilator 40 or the living body, the needle portion 16 can be restrained from protruding due to shrinking of the cover portion 30 by a frictional force. When the guide wire 10 is further pressed after the puncture portion 15 penetrates the oval fossa O and reaches the left atrium L, the cover portion 30 penetrates the hole formed. In this instance, since the wire member 31 of the coarse pitch portion 33, the dense pitch portion 34, and the curved portion 35 is arranged such that adjacent loops of the wire member 31 have no gap therebetween, a further axial shrinkage cannot occur. Therefore, when the guide wire 10 is pressed forward, the cover portion 30 penetrates the formed hole subsequentially to the puncture portion 15. When the distal end of the cover portion 30 penetrates the hole, the cover portion 30 in an elastically shrunk state is extended axially by its own restoring force, as depicted in FIG. 7(B). As a result, the puncture portion 15 is accommodated in the cover portion 30 (step S16). The puncture portion 15 is accommodated in the dense pitch portion 34. The curved portion 35 is restored into the original bent shape and is located on the distal side of the needle portion 16. The cover portion 30 accommodating the puncture portion 15 is protruding from the dilator 40 and, therefore, can flex freely. For this reason, the puncture portion 15 in the left atrium L is kept in the state of being accommodated in the cover portion 30. Therefore, the puncture portion 15 can be restrained from erroneously puncturing positions other than a desired position. Since the curved portion 35 is bent, the distal end of the guide wire 10 is not liable to collide against the living body tissue. Therefore, damage to the living body tissue can be restrained by the curved portion 35. The guide wire 10 is provided with the cover portion 30 at the outer periphery of the thin shaft portion 11. Therefore, by pressing the cover portion 30 into the hole formed by the needle portion 16, the hole can be smoothly expanded. For this reason, by using the cover portion 30, the guide wire 10 can form a hole of a suitable size in the oval fossa O. In the case where only a part of the puncture portion 15 is protruding from the dilator 40, the puncture portion 15 is supported by the dilator 40 and can exhibit a puncturing capability. Therefore, for securing safety, it is preferable that after the needle portion 16 penetrates the oval fossa O, the distal end of the cover portion 30 is located on the distal side relative to the puncture portion 15. Thereafter, when the whole part of the puncture portion 15 protrudes from the dilator 40, the puncture portion 15 loses the support by the dilator 40. As a result, that portion of the guide wire 10 which protrudes from the dilator 40 is bent flexibly, so that the puncture portion 15 does not exhibit the puncturing capability and safety can be secured.

When the puncture portion 15 penetrates the oval fossa O, a part of the tapered portion 42 of the dilator 40 having pressed the oval fossa O toward the left atrium L side enters into the hole opened in the oval fossa O. Note that a part of the tapered portion 42 may not enter into the hole in the oval fossa O.

When the puncture portion 15 penetrates the oval fossa O, the left atrium L communicates favorably with the spaces inside the dilator 40 and the inserter 60 through the through-hole 17 of the puncture portion 15, the gap between the cover portion 30 and the shaft portion 11, and the gap between adjacent loops of the wire member 31 constituting the cover portion 30. Therefore, by measuring the blood pressure through the port portion 66 of the inserter 60, it can be accurately confirmed that the puncture portion 15 has reached the left atrium L. In addition, a contrast medium can be released from the port portion 66 of the inserter 60 into the left atrium L via the through-hole 17 of the puncture portion 15.

Next, the sheath assembly 20 is moved toward the distal side, preceded by the guide wire 10. As a result, as illustrated in FIG. 8(A), the tapered portion 42 of the dilator 40 and the sheath tapered portion 53 of the outer sheath 50 pass through the oval fossa O while expanding the hole in the oval fossa O, and reach the left atrium L. In other words, the guide wire 10 guides the movement of the dilator 40 at least from the right atrium R to the oval fossa O side of the left atrium L. In this instance, since the tapered portion 42 and the sheath tapered portion 53 are decreasing in diameter toward the distal side, they can smoothly expand the hole in the oval fossa O (step S17). In addition, since the guide wire 10 is kept in the state of penetrating the oval fossa O, the dilator 40 and the outer sheath 50 can be easily pressed into the hole of the oval fossa O along the guide wire 10. Besides, since a part of the tapered portion 42 of the dilator 40 is entering into the hole of the oval fossa O when the puncture portion 15 punctures the oval fossa O, it is easy to press the dilator 40 and the outer sheath 50 into the hole of the oval fossa O. In addition, with the guide wire 10 preceding, the outer sheath 50 can be safely moved to the left atrial wall side (for example, to the vicinity of a pulmonary vein).

Subsequently, as depicted in FIG. 8(B), the inserter 60 and the dilator 40 are withdrawn to the outside of the body, leaving the outer sheath 50 and, if necessary, the guide wire 10 inside the body (step S18). The hole of the oval fossa O expanded by the dilator 40 is maintained by the outer sheath 50. When the dilator 40 is withdrawn from the outer sheath 50, the valve body 55 is closed, whereby leaking of blood and mixing of air or the like into the blood vessel can be restrained. Thereafter, a second medical device is inserted from the proximal side of the outer sheath 50 through the valve body 55. The second medical device may be, for example, a therapeutic device such as an ablation catheter, an image diagnostic device, an electrode catheter, or the like. As a result, using the outer sheath 50 penetrating the oval fossa O, the second medical device can be inserted to a desired position (for example, left atrium L, right atrium R, left auricle, and mitral valve) (step S19). After the second medical device is used (step S20), the second medical device and the outer sheath 50 are withdrawn to the outside of the body (step S21), whereon the hole of the oval fossa O shrinks. As a result, the procedure is completed.

Before the dilator 40 and the outer sheath 50 are pressed into the hole of the oval fossa O, the dilator 40 may be detached from the oval fossa O, as depicted in FIG. 9, due to a reaction at the time of pressing the guide wire 10 forward. Forces received by the dilator 40 from beating of the heart and blood flow in the heart may also cause the dilator 40 to be detached from the oval fossa O. In this instance, since the coarse pitch portion 33 of the cover portion 30 is located on the proximal side relative to the dense pitch portion 34, the coarse pitch portion 33 is not liable to be exposed to the distal side relative of the dilator 40. In other words, that part of the cover portion 30 which is exposed to the distal side relative to the dilator 40 is highly possibly the dense pitch portion 34. Since the dense pitch portion 34 is less liable to be bent than the coarse pitch portion 33, bending of the guide wire 10 between the dilator 40 and the oval fossa O can be restrained. Therefore, even when the dilator 40 is detached from the oval fossa O, it is easy to press the dilator 40 and the outer sheath 50 into the hole of the oval fossa O along the guide wire 10. In addition, since the dense pitch portion 34 that has a smaller gap between adjacent loops of the wire member 31 than that of the coarse pitch portion 33 is covering the puncture portion 15, the puncture portion 15 can be restrained from protruding via the gap between adjacent loops of the wire member 31, so that safety is high.

As has been described above, the guide wire 10 in the present embodiment is a guide wire 10 for guiding the pipe-shaped dilator 40 (elongate body) to be inserted in a living body. The guide wire 10 includes the elongate shaft portion 11 having flexibility, the puncture portion 15 that is fixed (disposed) to a distal portion of the shaft portion 11 and that forms a hole in the living body tissue, and the cover portion 30 that is elastically deformable in the axial direction and that covers the puncture portion 15. When the cover portion 30 is exposed from the dilator 40, the cover portion 30 covers the puncture portion 15, and the cover portion 30 is bent.

In the guide wire 10 configured as above, in the state in which the cover portion 30 is located in the inside of the dilator 40, only flexure of a distal portion of the shaft portion 11 is limited by the dilator 40 and the cover portion 30, so that a hole can be formed in the oval fossa O by the puncture portion 15. In this way, the guide wire 10 can form a hole in the living body tissue while functioning as a guide wire for guiding the dilator 40 and the ablation catheter. Besides, the guide wire 10 has high workability since it is unnecessary to use a separate device having a puncturing function. In addition, when the cover portion 30 is exposed from the elongate body such as the dilator 40, the cover portion 30 covers the puncture portion 15, and the cover portion 30 is bent on the distal side of the puncture portion 15. Therefore, the guide wire 10 restrains erroneous puncture by the puncture portion 15, whereby high safety can be obtained.

In addition, the cover portion 30 is deformable into a rectilinear shape by being accommodated in the dilator 40. As a result, the cover portion 30 becomes rectilinear in shape in the inside of the dilator 40 and becomes easily shrinkable in the axial direction, so that it becomes easy to expose the puncture portion 15.

Besides, the cover portion 30 is fixed to the shaft portion 11. The cover portion 30, in the state of being accommodated in the inside of the dilator 40, receives a proximally directed force at the distal end thereof to elastically shrink in the axial direction, thereby exposing the puncture portion 15. Further, the cover portion 30, in the state of being located outside the dilator 40, receives a proximally directed force at the distal end thereof to flex together with a distal portion of the shaft portion 11, thereby maintaining the state of accommodating the puncture portion 15. Therefore, in the state in which the cover portion 30 of the guide wire 10 is exposed to the outside of the dilator 40, even when a proximally directed force is received at the distal end of the cover portion 30, the cover portion 30 flexes to maintain the state in which the puncture portion 15 is accommodated in the cover portion 30. Therefore, the guide wire 10 can guide the dilator 40 or the like to a desired position while preventing the puncture portion 15 from protruding from the cover portion 30 and while maintaining safety. Besides, when the distal end of the cover portion 30 attaches to the living body tissue and receives a proximally directed force, in the state in which the cover portion 30 is accommodated in the dilator 40, the cover portion 30 elastically shrinks in the axial direction, and the puncture portion 15 is exposed. Therefore, with the cover portion 30 being accommodated in the dilator 40 or the like, a hole can be formed in the living body tissue by the puncture portion 15. After the protruding puncture portion 15 penetrates the living body tissue, the cover portion 30 returns to its original shape by its own restoring force to accommodate the puncture portion 15. In addition, the guide wire 10 can expand the hole formed by the puncture portion 15, by the cover portion 30 accommodating the puncture portion 15, whereby a hole of a suitable size can be formed in the living body tissue.

Besides, the cover portion 30 has the curved portion 35 which is curved in a natural state. As a result, in a state in which the cover portion 30 is located in the outside of the dilator 40, a force for shrinking the cover portion 30 in the axial direction is not easily exerted on the distal end of the cover portion 30. Therefore, the puncture portion 15 does not protrude from the cover portion 30, erroneous puncture by the puncture portion 15 is restrained, and high safety is obtained. Besides, in the living body, the distal end of the cover portion 30 is not liable to collide against the living body tissue, so that damage to the living body tissue can be restrained.

In addition, the proximal end of the curved portion 35 is located on the distal side of the puncture portion 15. This ensures that the curved portion 35 does not easily shrink in the axial direction even when receiving a force in a state of being located outside the dilator 40. Therefore, the state in which the cover portion 30 covers the puncture portion 15 can be maintained, and high safety is obtained. In addition, in the living body, the distal end of the curved portion 35 is not liable to collide against the living body tissue, so that damage to the living body tissue can be restrained.

Besides, the puncture portion 15 has the through-hole 17 penetrating in the axial direction. As a result, when the puncture portion 15 penetrates the oval fossa O, for example, a contrast medium or the like can be supplied into the living body through the puncture portion 15. Besides, when the puncture portion 15 penetrates the oval fossa O, the pressure in the living body can be measured via the through-hole 17, whereby the presence or absence of penetration can be grasped accurately.

In addition, the cover portion 30 is a member having a helical shape, and at least a part of the cover portion 30 has a gap between adjacent loops of the wire member 31 aligned in the axial direction while forming a helix. As a result, the cover portion 30 can expose the puncture portion 15 by shrinking in the axial direction in such a manner as to reduce the gap between adjacent loops of the wire member 31, from the state of covering the puncture portion 15.

Besides, the cover portion 30 includes the coarse pitch portion 33 that has a gap between adjacent loops of the wire member 31 aligned in the axial direction while forming a helix, and the dense pitch portion 34 which is located on the distal side relative to the coarse pitch portion 33 and in which a pitch distance of the wire member 31 aligned in the axial direction while forming a helix is shorter than that in the coarse pitch portion 33. As a result, the coarse pitch portion 33 can shrink in the axial direction such as to reduce the gap between adjacent loops of the wire member 31, from the state of covering the puncture portion 15, to expose the puncture portion 15. In addition, the dense pitch portion 34 does not easily shrink in the axial direction, and, therefore, can effectively transmit forces in the axial direction and permits effective puncture. Incidentally, after a hole is formed in the living body tissue by the guide wire 10 and before the dilator 40 is pressed into the hole, the dilator 40 may be detached from the living body tissue. In this instance, since the coarse pitch portion 33 of the cover portion 30 is located on the proximal side relative to the dense pitch portion 34, the coarse pitch portion 33 is not liable to be exposed to the distal side relative to the dilator 40. In other words, that part of the cover portion 30 which is exposed to the distal side relative to the dilator 40 is highly possibly the dense pitch portion 34. Since the dense pitch portion 34 is less liable to be bent than the coarse pitch portion 33, bending of the guide wire 10 between the dilator 40 and the living body tissue can be restrained. Therefore, even when the dilator 40 is detached from the living body tissue, it is easy to press the dilator 40 into the hole of the living body tissue along the guide wire 10.

In addition, a distal portion of the puncture portion 15 has the inclined surface 18 inclined relative to the center axis of the puncture portion 15, the sharp needle portion 16 of the puncture portion 15 is located at the distal end of the inclined surface 18, and the direction in which the curved portion 35 is curved coincides with the direction in which the needle portion 16 is located in the inclined surface 18. As a result, when the puncture portion 15 is moved in the cover portion 30 toward the distal side, the needle portion 16 can be restrained from making contact with the inner surface of the curved portion 35. Therefore, the needle portion 16 can be restrained from damaging the curved portion 35, and the needle portion 16 can be restrained from unintentionally protruding from the curved portion 35 to damage the living body tissue.

Besides, the medical device 1 according to the present embodiment is a medical device 1 for forming a hole in the oval fossa O (living body tissue) in the living body, and includes the pipe-shaped dilator 40 (elongate body) to be inserted in the living body, and the guide wire 10 insertable in the dilator 40. The guide wire 10 includes the elongate shaft portion 11 having flexibility, the puncture portion 15 that is fixed (disposed) to a distal portion of the shaft portion 11 and that forms a hole in the living body tissue, and the cover portion 30 that is elastically deformable in the axial direction and that covers the puncture portion 15. When the cover portion 30 is exposed from the dilator 40, the cover portion 30 covers the puncture portion 15, and the cover portion 30 is bent.

The medical device 1 configured as above has the dilator 40 (elongate body) in which to insert the aforementioned guide wire 10, and, therefore, the cover portion 30 of the guide wire 10 can be accommodated in the dilator 40. In a state in which the cover portion 30 is accommodated in the dilator 40, a distal-side end portion of the cover portion 30 is brought into contact with the oval fossa O by the dilator 40 and the cover portion 30, and the cover portion 30 shrinks in the axial direction to expose the puncture portion 15, whereby a hole can be formed in the oval fossa O by the puncture portion 15. In addition, when the cover portion 30 penetrates the oval fossa O and is exposed from the dilator 40, the cover portion 30 covers the puncture portion 15, and the cover portion 30 is bent in such a manner as not to expose the puncture portion 15. Therefore, the medical device 1 can restrain erroneous puncture by the puncture portion 15, so that high safety is obtained. The cover portion 30 may not have the curved portion. In this case, the cover portion 30 may be rectilinear in shape, in the state in which the cover portion 30 is accommodated in the dilator 40 and in the state in which the cover portion 30 covers the puncture portion 15 having penetrated the oval fossa O. Further, the cover portion 30 may have the same shape, both in the state of being accommodated in the dilator 40 and in the state of covering the puncture portion 15.

In addition, the present disclosure also includes a treatment method (therapeutic method), which is not part of the invention, for forming a hole in an oval fossa O (living body tissue) in a living body by use of the aforementioned medical device 1. The treatment method, which is not part of the invention, includes a step of accommodating the puncture portion 15 and the cover portion 30 of the guide wire 10 in the dilator 40 and putting the distal end of the dilator 40 into contact with the living body tissue (step S13), a step of pressing the distal end of the cover portion 30 against the oval fossa O and forming a hole in the oval fossa O by the puncture portion 15 exposed from the cover portion 30 (step S15), a step of pressing the cover portion 30 into the hole of the oval fossa O subsequentially to the puncture portion 15, extending the cover portion 30 in the axial direction to accommodate the puncture portion 15 in the cover portion 30, disposing the distal portion of the cover portion 30 on the distal side of the puncture portion 15, and curving at least one of the cover portion 30 and the shaft portion 11 (step S16), and a step of moving the dilator 40 along the guide wire 10 (step S17).

In the treatment method, which is not part of the invention, configured as above, the puncture portion 15 and the cover portion 30 of the guide wire 10 are accommodated in the dilator 40, and the distal end of the cover portion 30 is pressed against the living body tissue to form a hole in the living body tissue by the puncture portion 15. Therefore, the treatment method, which is not part of the invention, has high workability since it is unnecessary to use a separate device having a puncturing function. Besides, in a state in which the cover portion 30 is located in the outside of the dilator 40, by receiving a proximally directed force at the distal end of the cover portion 30, a distal portion of the shaft portion 11 flexes together with the cover portion 30. On the distal side of the puncture portion 15, a bent portion of the cover portion 30 is disposed. Therefore, according to the treatment method, which is not part of the invention, erroneous puncture by the puncture portion 15 can be restrained, and safety can be secured. In addition, according to the treatment method, which is not part of the invention, the hole formed by the puncture portion 15 can be expanded by the cover portion 30 accommodating the puncture portion 15, whereby a hole of a suitable size can be formed in the living body tissue.

Incidentally, the present invention is not limited to the aforementioned embodiment, and various modifications can be made by those skilled in the art within the technical thought of the invention. For example, the aforementioned medical device may be used for puncturing living body tissue in a living body other than the oval fossa. Besides, the medical device may not be used for expanding the hole in the living body tissue. For example, the medical device may be used as an injection tool for puncturing the living body tissue (for example, cardiac muscle and organ) in a living body and injecting a chemical agent, a contrast medium, a physiological saline, or the like. The chemical agent may include, for example, cells to be injected to cardiac muscle. The elongate body in which the needle portion 16 is accommodated may be pressed into or may not be pressed into the hole formed in the living body tissue.

In addition, the cover portion 30 may not be formed of a single wire member 31 but may be formed of a plurality of wire members. Therefore, the cover portion may be formed of a multi-coil spring or mesh. Besides, the cover portion may be a flexible resin tube.

Besides, while the dilator 40 and the inserter 60 are separate bodies, they may be integral with each other. In addition, if the dilator 40 (elongate body) is present, the inserter 60 may not be provided.

In addition, as in a first modification depicted in FIG. 10, a dense pitch portion 82 may be provided on the proximal side of a coarse pitch portion 83. Note that the parts having functions similar to those of the parts in the aforementioned embodiment are denote by the same reference symbols as used above, and descriptions thereof are omitted. A cover portion 81 of a guide wire 80 includes the dense pitch portion 82, the coarse pitch portion 83 located on the distal side of the dense pitch portion 82, and a curved portion 35 located on the distal side relative to the coarse pitch portion 83. The dense pitch portion 82 has a wire member 31 in a helical shape with no gap between adjacent loops thereof. The dense pitch portion 82 is located on the most proximal side of the cover portion 81. The proximal end of the dense pitch portion 82 is fixed to a shaft portion 11. The coarse pitch portion 83 has the wire member 31 in a helical shape with a gap between adjacent loops thereof. A proximal portion of the cover portion 81 may be formed with a plurality of circulation holes 84. The circulation holes 84 are, for example, formed at an edge portion of the wire member 31 aligned in the axial direction, but the form of the holes is not limited. With the circulation holes 84 formed in the proximal portion of the cover portion 81, it is easy for a fluid to be circulated between an inner peripheral surface and an outer peripheral surface of the cover portion 81 even if the pitch of the helix of the wire member 31 is small (even if there is no gap between adjacent loops of the wire member 31 aligned in the axial direction). As a result, a contrast medium or blood can be favorably circulated through a through-hole 17 in a puncture portion 15, the gap between the cover portion 81 and the shaft portion 11, and the circulation holes 84. Since the wire member 31 at the proximal portion of the cover portion 81 is disposed densely with no gap between adjacent loops thereof, forces can be effectively transmitted in the axial direction in the inside of the dilator 40. Note that the position of the cover portion 81 where the circulation holes 84 are formed is not limited to the proximal portion.

Besides, as in a second modification depicted in FIG. 11(A), a cover portion 91 of a guide wire 90 may have a curved portion 92 which is a pipe-shaped member. Note that the parts having functions similar to those of the parts in the aforementioned embodiment are denoted by the same reference symbols as used above, and descriptions thereof are omitted. The curved portion 92 is a resin-made pipe-shaped member which is flexible and elastically deformable. The curved portion 92 is located at a distal portion of a coarse pitch portion 33 fixed to a shaft portion 11 by a fixing portion 36. The curved portion 92 is located from a distal portion of the coarse pitch portion 33 to the distal end of the cover portion 91. The curved portion 92 may be a part of the portion ranging between the distal portion of the coarse pitch portion 33 to the distal end of the cover portion 91. In that case, the curved portion 92 is located in the state of being interposed between the coarse pitch portion 33 and a part of written challenge of the cover portion 91. A proximal portion of the curved portion 92 is covering a puncture portion 15.

When the guide wire 90 is located in the inside of an elongate body such as a dilator 40, the cover portion 91 is deformed into a rectilinear shape. In this state, when a proximally directed force acts on the distal end of the cover portion 91, the coarse pitch portion 33 shrinks in the axial direction. As a result, as depicted in FIG. 11(B), a needle portion 16 is exposed from the curved portion 92 and is able to puncture the living body tissue. When the distal end of the cover portion 91 passes through a hole of the living body tissue sequentially to the needle portion 16, the coarse pitch portion 33 extends in the axial direction by its own restoring force, as depicted in FIG. 11(A). As a result, the curved portion 92 is restored into a shape of covering the needle portion 16 and being bent. Therefore, according to the guide wire 90, erroneous puncture by the needle portion 16 for puncture can be restrained by the cover portion 91 having the curved portion 92.

In addition, as in a third modification depicted in FIG. 12(A), a cover portion 111 of a guide wire 110 may include a distal cover portion 112 extending radially outward by shrinking in the axial direction in the inside of a dilator 40, a pipe-shaped proximal cover portion 114, and a pipe-shaped curved portion 115. The distal cover portion 112 is formed with a plurality of slits 113 forming helices. The slits 113 penetrate between an inner peripheral surface and an outer peripheral surface of the distal cover portion 112. The proximal cover portion 114 is an elastically deformable pipe-shaped member and is formed, in a proximal portion thereof, with a plurality of circulation holes 116. With the circulation holes 116 formed in the proximal portion of the cover portion 111, a fluid can be easily circulated between the inside and the outside of the cover portion 111. As a result, a contrast medium and blood can be favorably circulated through a through-hole 17 in a puncture portion 15, the gap between the cover portion 111 and a shaft portion 11, and the circulation holes 116. Note that the position of the cover portion 111 where the circulation holes 116 are formed is not limited to the proximal portion. When the guide wire 110 is located in the inside of an elongate body such as a dilator 40, the cover portion 111 is deformed into a rectilinear shape. In this state, when a proximally directed force acts on the distal end of the cover portion 111, the slits 113 are widened and are expanded in the radial direction within the range of a space inside the dilator 40 as depicted in FIG. 12(B). Therefore, when a proximally directed force is received at the distal end of the cover portion 111, the cover portion 111 is expanded radially outward while shrinking in the axial direction. For this reason, after a needle portion 16 penetrates the living body tissue, by the passage of the distal cover portion 112 expanded in the radial direction through a hole in the living body tissue, the hole can be expanded largely. When the distal end of the cover portion 111 passes through the hole of the living body tissue subsequentially to the needle portion 16, the distal cover portion 112 extends in the axial direction by its own restoring force. As a result, the curved portion 115 is restored into the shape of covering the needle portion 16 and being curved. Therefore, according to the guide wire 110, erroneous puncture by the needle portion 16 for puncture can be restrained by the cover portion 111 having the curved portion 115.

Besides, as in a fourth modification depicted in FIG. 13(A), a cover portion 140 of a guide wire 130 may have a distal pipe 141 on the distal side of a curved portion 35. Note that the parts having functions similar to those of the parts in the aforementioned embodiment are denoted by the same reference symbols as used above, and descriptions thereof are omitted.

The distal pipe 141 is a circular pipe fixed to a distal portion of the curved portion 35. The distal pipe 141 is higher in bending rigidity than a coarse pitch portion 33, a dense pitch portion 34, and the curved portion 35 that are formed into a helical shape.

The constituent material of the distal pipe 141 preferably has a certain extent of rigidity. Examples of the material include shape memory alloys to which a shape memory effect or superelasticity is imparted by a heat treatment, metals such as stainless steel, tantalum, titanium, platinum, gold, and tungsten, polyolefins such as polyethylene and polypropylene, polyamides, polyesters such as polyethylene terephthalate, fluoro-polymers such as PTFE (polytetrafluoroethylene) and ETFE (ethylene-tetrafluoroethylene copolymer), PEEK (polyether ether ketone), and polyimides.

When the guide wire 130 is located in the inside of an elongate body such as a dilator 40, the cover portion 140 is deformed into a rectilinear shape. In this state, when the guide wire 130 is moved toward the distal side relative to the dilator 40 and the distal end of the guide wire 130 comes into contact with the living body tissue, a proximally directed force acts on the distal end of the cover portion 140 located at the most distal position of the guide wire 130. As a result, as depicted in FIG. 13(B), a gap between adjacent loops of a wire member 31 of the coarse pitch portion 33 is reduced, and the coarse pitch portion 33 shrinks in the axial direction. As a result, the dense pitch portion 34 of the cover portion 140 and the distal pipe 141 are moved toward the proximal side, and a needle portion 16 of a puncture portion 15 accommodated in the cover portion 140 is exposed. In this instance, a proximal portion of the puncture portion 15 is surrounded by the distal pipe 141 which is high in rigidity. Therefore, the puncture portion 15 is stabilized in posture by being supported by the distal pipe 141 of the cover portion 140. For this reason, the guide wire 130 can smoothly form a hole in the living body tissue by the puncture portion 15. When the distal end of the cover portion 140 passes through the hole of the living body tissue subsequentially to the needle portion 16, the coarse pitch portion 33 extends in the axial direction by its own restoring force. As a result, the cover portion 140 covers the needle portion 16 and is restored into the curved shape. Therefore, according to the guide wire 130, erroneous puncture by the needle portion 16 for puncture can be restrained by the cover portion 140 having the curved portion 35.

In addition, as in a fifth modification depicted in FIG. 14, a cover portion 160 of a guide wire 150 may be provided with an inclined part 161 on the distal side of a curved portion 35. Note that the parts having functions similar to those of the parts in the aforementioned embodiment are denoted by the same reference symbols as used above, and descriptions thereof are omitted.

The inclined part 161 is provided on the distal side of the curved portion 35. A distal-side end portion of the inclined part 161 is inclined relative to the center axis of the cover portion 160. In a state in which the curved portion 35 is deformed into a rectilinear shape, the inclination direction of the inclined part 161 relative to the center axis of the cover portion 160 preferably coincide with, but may not coincide with, the inclination direction of an inclined surface 18 relative to the center axis of a puncture portion 15. An inclination angle α4 of the inclined part 161 relative to the center axis of the cover portion 160 is greater than an inclination angle α1 of the inclined surface 18 relative to the center axis of the puncture portion 15. Therefore, an end portion of the inclined part 161 does not have a puncturing capability like that of the needle portion 16. The inclined part 161 is formed smoothly by a method in which a gap between adjacent loops of a wire member 31 is filled up by brazing, or adjacent loops of the wire member 31 are put into contact with one another and welded to one another, after which the resultant body is cut to be inclined, and an outer edge portion thereof is subjected to curved surface processing.

When the guide wire 150 is located in the inside of an elongate body such as the dilator 40, the cover portion 160 is deformed into a rectilinear shape. In this state, when the guide wire 150 is moved toward the distal side relative to the dilator 40 and the distal end of the guide wire 150 comes into contact with the living body tissue, a proximally directed force acts on the distal end of the cover portion 160 located at the most distal position of the guide wire 150. As a result, as illustrated in FIG. 15(A), a gap between adjacent loops of the wire member 31 of the coarse pitch portion 33 is reduced, and the coarse pitch portion 33 shrinks in the axial direction. Consequently, the dense pitch portion 34 and the curved portion 35 of the cover portion 160 are moved toward the proximal side, and the needle portion 16 of the puncture portion 15 accommodated in the cover portion 160 is exposed. In this instance, a proximal portion of the puncture portion 15 is surrounded by the inclined part 161 of the cover portion 160 where adjacent loops of the wire member 31 are fixed to one another. Therefore, the puncture portion 15 is stabilized in posture by being supported by the inclined part 161 which is high in rigidity. For this reason, the guide wire 150 can smoothly form a hole in the living body tissue by the puncture portion 15.

The shrunk cover portion 160 is pressed into the hole formed by the puncture portion 15, subsequentially to the puncture portion 15. Since the inclined part 161 is provided at the distal end of the cover portion 160, the cover portion 160 can smoothly pass through the hole of the living body tissue with a small force. In this instance, the distal end of the inclined part 161 of the cover portion 160 in the shrunk state coincides with, or is located on the proximal side relative to, the proximal end of the inclined surface 18 of the puncture portion 15. As a result, the inclined part 161 reaches the living body tissue after the inclined surface 18 of the puncture portion 15 has passed through the living body tissue. Therefore, the cover portion 160 can expand the hole of the living body tissue smoothly with a small force. In addition, since the inclination direction of the inclined part 161 coincides with the inclination direction of the inclined surface 18, the cover portion 160 can smoothly expand the hole of the living body tissue formed by the puncture portion 15. When the distal end of the cover portion 160 passes through the hole of the living body tissue subsequentially to the needle portion 16, the coarse pitch portion 33 extends in the axial direction by its own restoring force. As a result, the cover portion 160 covers the needle portion 16 and is restored into a curved shape. Therefore, according to the guide wire 150, erroneous puncture by the needle portion 16 for puncture can be restrained by the cover portion 160 having the curved portion 35. Incidentally, as in a sixth modification depicted in FIG. 15(B), the distal end of the inclined part 161 of the cover portion 160 in a shrunk state may be located on the proximal side relative to the distal end of the inclined surface 18 of the puncture portion 15 and located on the distal side relative to the proximal end of the inclined surface 18. In this case, the amount of exposure of the sharp needle portion 16 from the cover portion 160 can be suppressed, a moving distance until the shrunk cover portion 160 again entirely covers the needle portion 16 can be shortened, and safety is enhanced. Note that while the inclined part 161 of the cover portion 160 is formed from the wire member 31, the inclined part may be formed, for example, by fixing a separate member to the wire member 31.

In addition, as in a seventh modification depicted in FIG. 16(A), a cover portion 180 of a guide wire 170 may be provided with a first stopper 181 and a second stopper 182. Note that the parts having functions similar to those of the parts in the aforementioned embodiment are denoted by the same reference symbols as used above, and descriptions thereof are omitted.

The cover portion 180 may be provided, at an inner peripheral surface of a coarse pitch portion 33, with at least one first stopper 181 making contact with the proximal end of a puncture portion 185. The first stopper 181 restricts the puncture portion 185 from moving toward the proximal side relative to the first stopper 181. Therefore, for example, when the cover portion 180 is pulled toward the distal side at the time of withdrawing the guide wire 170 to the outside of the body, the first stopper 181 makes contact with the proximal end of the puncture portion 185. Therefore, the pitch distance of the wire member 31 of the cover portion 180 can be restrained from being enlarged excessively. Consequently, a needle portion 16 can be restrained from protruding through a gap between adjacent loops of the wire member 31, the cover portion 180 can be restrained from being detached from the guide wire 170, and high safety can be obtained.

The cover portion 180 is provided, at the distal end thereof, with the second stopper 182 decreasing in inner diameter. The second stopper 182 restricts the whole body of the puncture portion 185 from moving toward the distal side relative to the second stopper 182 when the cover portion 180 shrinks, as illustrated in FIG. 16(B). Therefore, even if the fixation of the puncture portion 185 and a shaft portion 11 by a needle fixing portion 19 should be canceled, the puncture portion 185 can be maintained inside the cover portion 180 and can be restrained from remaining in the body.

A proximal portion of the puncture portion 185 has been subjected to spiral cutting and is formed in a spiral shape. As a result, the proximal portion of the puncture portion 185 is flexible and easily bendable. Therefore, bending rigidity smoothly varies between the puncture portion 185 and the shaft portion 11 in the axial direction, so that kinking hardly occurs. Note that, for making the proximal portion of the puncture portion flexible, a flexible tube may be connected instead of applying spiral cutting.

Besides, as in an eighth modification depicted in FIG. 17(A), a cover portion 200 of a guide wire 190 may include a coarse pitch portion 201, a dense pitch portion 202 on the distal side relative to the coarse pitch portion 201, and a distal pipe 203 on the distal side relative to the dense pitch portion 202. Note that the parts having functions similar to those of the parts in the aforementioned embodiment are denoted by the same reference symbols as used above, and descriptions thereof are omitted.

The coarse pitch portion 201 is longer in pitch distance of a helix than the dense pitch portion 202. The proximal side of the cover portion 200, that is, the proximal end of the coarse pitch portion 201, is fixed to a shaft portion 11. The coarse pitch portion 201 can shrink in the axial direction in such a manner that a gap between adjacent loops of a wire member 31 aligned in the axial direction is reduced. The coarse pitch portion 201 and the dense pitch portion 202 surround a part of the shaft portion 11. The inner diameter of the dense pitch portion 202 is smaller than the outer diameter of a puncture portion 15. The distal end of the dense pitch portion 202 has a step at an inner peripheral surface thereof, and this step is a first stopper 204 which makes contact with the proximal end of the puncture portion 15. The first stopper 204 restricts the puncture portion 15 from moving toward the proximal side relative to the first stopper 204. The distal pipe 203 is a circular pipe fixed to a distal portion of the dense pitch portion 202. The distal pipe 203 has a curved portion 206 curved to the distal side. The distal pipe 203 is a pipe-shaped member which is flexible and elastically deformable. The distal pipe 203 is higher in bending rigidity than the coarse pitch portion 201 and the dense pitch portion 202. The distal pipe 203 surrounds the puncture portion 15 and a part of the shaft portion 11. The outer diameter of the distal pipe 203 is substantially coincident with the outer diameter of the dense pitch portion 202. The inner diameter of the distal pipe 203 is greater than the inner diameter of the dense pitch portion 202 and is greater than the outer diameter of the puncture portion 15. Therefore, the distal pipe 203 is thinner than the dense pitch portion 202. The distal pipe 203 is provided, at an inner peripheral surface of a distal portion thereof, with a second stopper 205 protruding radially inward. The second stopper 205 is located on the side opposite to a position where a needle portion 16 of the puncture portion 15 is provided, in the circumferential direction. The puncture portion 15 is provided, in an inner peripheral surface of a distal portion thereof, with a groove part 15A extending proximally from the distal end. The groove part 15A is located on the proximal side of the second stopper 205. The second stopper 205 can enter the groove part 15A from the distal side.

When the cover portion 200 is pulled toward the distal side at the time of, for example, withdrawing the guide wire 190 to the outside of the body, the first stopper 204 comes into contact with the proximal end of the puncture portion 15, as illustrated in FIG. 17(A). Therefore, the pitch distance of the wire member 31 of the cover portion 200 can be restrained from being enlarged excessively. For this reason, the needle portion 16 can be restrained from protruding through a gap between adjacent loops of the wire member 31, the cover portion 200 can be restrained from being detached from the guide wire 190, and high safety is obtained. In addition, since the distal pipe 203 is thin, the outer diameter of the puncture portion 15 can be enlarged, and a hole can be formed largely in the living body tissue by the puncture portion 15. Besides, since the distal pipe 203 is thin, it is possible, after puncture by the puncture portion 15, to insert the distal pipe 203 into a hole of the living body tissue smoothly with small resistance. When the cover portion 200 shrinks and the needle portion 16 of the puncture portion 15 protrudes from the distal pipe 203, the second stopper 205 enters the groove part 15A, as depicted in FIG. 17(B). Upon reaching the most proximal side of the groove part 15A, the second stopper 205 abuts on an end surface of the groove part 15A. As a result, the cover portion 200 can be restrained from retracting excessively relative to the puncture portion 15, and a suitable positional relation between the cover portion 200 and the puncture portion 15 can be maintained. Therefore, the living body tissue can be punctured while maintaining the cover portion 200 at a desired position relative to the puncture portion 15.

In addition, as in a ninth modification depicted in FIG. 18, a guide wire 210 may include a needle fixing portion 211 protruding beyond an inclined surface 18 of a puncture portion 15. Note that the parts having functions similar to those of the parts in the aforementioned embodiment are denoted by the same reference symbols as used above, and descriptions thereof are omitted.

The distal end of a shaft distal portion 14 is fixed to an inner peripheral surface of the puncture portion 15 by welding, adhesion, or the like by the needle fixing portion 211. The needle fixing portion 211 protrudes to the outside beyond a surface where the inclined surface 18 of the puncture portion 15 is located. In other words, the needle fixing portion 211 protrudes to the outside beyond a surface M where a ring-shaped inner edge part 17A of an opening of a through-hole 17 in the inclined surface 18 is located. The needle fixing portion 211 is located on the proximal side relative to the distal end of a needle portion 16. The shape of a surface of a protruding part 212 protruding beyond the inclined surface 18 of the needle fixing portion 211 is not particularly limited but is preferably free of a corner and smooth. The needle fixing portion 211 including the protruding part 212 is provided such as not to close the through-hole 17 of the puncture portion 15.

When the guide wire 210 is protruded from a dilator 40 and the distal end of the guide wire 210 comes into contact with the living body tissue, a proximally directed force acts on the distal end of a cover portion 30 located at the most distal position of the guide wire 210. As a result, a gap between adjacent loops of a wire member 31 of a coarse pitch portion 33 is reduced, and the coarse pitch portion 33 shrinks in the axial direction. Consequently, a dense pitch portion 34 and a curved portion 35 of the cover portion 30 are moved toward the proximal side, and the needle portion 16 is exposed from the cover portion 30. The needle portion 16 exposed from the cover portion 30 forms a hole in the living body tissue. In this instance, the protruding part 212 of the needle fixing portion 211 protrudes to the outside from the surface where the inclined surface 18 of the puncture portion 15 is located. Therefore, when the puncture portion 15 passes through the hole of the living body tissue, the protruding part 212 expands the hole formed by the needle portion 16. For this reason, the resistance at the time when the distal end of the cover portion 30 passes through the hole of the living body tissue while expanding the hole subsequentially to the puncture portion 15 is reduced. Consequently, the distal end of the cover portion 30 can smoothly pass through the hole of the living body tissue while expanding the hole with small resistance. If the surface of the protruding part 212 protruding from the inclined surface 18 of the needle fixing portion 211 is free of a corner and smooth, the protruding part 212 can more smoothly expand the hole formed by the needle portion 16. Note that, as in a tenth modification depicted in FIG. 19, a protruding part 231 may be a part of a distal portion of a shaft portion 230 of a guide wire 220. The protruding part 231 is fixed to an inner peripheral surface of a puncture portion 15 by welding, adhesion, or the like. The protruding part 231 protrudes to the outside from a surface where an inclined surface 18 of the puncture portion 15 is located. Note that the protruding part protruding to the outside beyond the inclined surface 18 may not be provided at a part where a shaft portion 11 and the puncture portion 15 are joined. For example, the protruding part may be a part of the puncture portion 15.

Besides, as in an eleventh modification depicted in FIG. 20, a guide wire 240 may include first markers 241 and 242 and a second marker 251. Note that the parts having functions similar to those of the parts in the aforementioned embodiment are denoted by the same reference symbols as used above, and descriptions thereof are omitted.

The first markers 241 and 242 are markers for grasping the position of a cover portion 30 inserted in a living body on a visual basis by use of X-ray. The first marker 241 is a wire member including an X-ray contrast material which is wound around a position of the shaft portion 250 where the proximal end of the cover portion 30 is fixed. The first marker 242 is a ring-shaped member including an X-ray contrast material which is fixed to the distal side relative to a curved portion 35 of the cover portion 30. Preferred examples of the X-ray contrast material include those including at least one metal or two or more alloys selected from the group consisting of gold, platinum, iridium, tungsten, their alloys, and silver-palladium alloys. With the first marker 241 provided, the operator can easily grasp the position of the cover portion 30 in the living body under radioscopy. Note that the position where the first marker 241 is provided is not limited to the proximal end of the cover portion 30. In addition, with the first marker 242 provided on the distal side relative to the curved portion 35 of the cover portion 30, the operator can easily grasp the position of the cover portion 30 moved relative to the needle portion 16 under radioscopy. Therefore, the operator can easily recognize whether or not the cover portion 30 is covering the needle portion 16, and can easily grasp the status of puncture. Note that the position where the first marker 242 is provided is not limited to the distal side relative to the curved portion 35 of the cover portion 30, but may be, for example, the curved portion 35. The second marker 251 is a graduation provided at regular intervals on a surface of a proximal portion of the shaft portion 250. With the second marker 251 provided, the operator can visually confirm, at hand, the moving distances of the guide wire 240 relative to a dilator 40 and an inserter 60. Therefore, the operator can grasp a pressing-in distance of the puncture portion 15 and can easily grasp the status of puncture (for example, that puncture is completed). Besides, with both the first markers 241 and 242 and the second marker 251 provided, the operator can confirm the status of puncture by the first markers 241 and 242 while adjusting the pressing-in distance of the puncture portion 15 by the second marker 251.

In addition, as in a twelfth modification depicted in FIG. 21, a shaft portion 270 of a guide wire 260 may have a curved portion 271, and a cover portion 280 may be rectilinear in shape. Note that the parts having functions similar to those of the parts in the aforementioned embodiment are denoted by the same reference symbols as used above, and descriptions thereof are omitted.

The distal end of the shaft portion 270 is fixed to a puncture portion 15 by a needle fixing portion 19. The curved portion 271 is provided at a shaft decreasing diameter portion 272 decreasing in diameter toward the distal side of the shaft portion 270. Note that the position where the curved portion 271 is provided is not limited to the shaft decreasing diameter portion 272 and may be, for example, a part where the outer diameter is constant. The curved portion 271 is located from a rectilinear portion of the shaft portion 270 to the distal end of the shaft portion 270. The curved portion 271 may be a part of a portion ranging from the rectilinear portion of the shaft portion 270 to the distal end of the shaft portion 270. In that case, the curved portion 271 is interposed between the rectilinear portions of the shaft portion 270. In addition, the curved portion 271 may be interposed between the rectilinear portion of the shaft portion 270 on the proximal side relative to the curved portion 271 and a proximal portion of the cover portion 280 on the distal side relative to the curved portion 271. The cover portion 280 includes a coarse pitch portion 281 having a long pitch distance of a helix. The coarse pitch portion 281 is formed of a wire member 31 in a helical shape with a gap between adjacent loops thereof. The cover portion 280 is fixed to a proximal portion of the puncture portion 15 by a fixing portion 282 located at the proximal end of the cover portion 280. Note that the proximal end of the cover portion 280 may be located on the proximal side relative to the fixing portion 282.

The guide wire 260 is accommodated in a dilator 40, with the curved portion 271 in a rectilinearly deformed state. When the guide wire 260 is moved toward the distal side in the inside of the dilator 40, the distal end of the guide wire 260 comes into contact with the living body tissue. Therefore, a proximally directed force acts on the distal end of the cover portion 280 located at the most distal position of the guide wire 260. As a result, the gap between adjacent loops of the wire member 31 of the coarse pitch portion 281 is reduced, and the coarse pitch portion 281 shrinks in the axial direction. Consequently, a distal portion of the cover portion 280 is moved toward the proximal side, and a needle portion 16 is exposed from the cover portion 280. The needle portion 16 exposed from the cover portion 280 forms a hole in the living body tissue. When the distal end of the cover portion 280 passes through the hole of the living body tissue subsequentially to the needle portion 16, the coarse pitch portion 281 extends in the axial direction by its own restoring force. As a result, the cover portion 280 covers the needle portion 16.

The cover portion 280 in the twelfth modification is fixed to the puncture portion 15. In other words, the cover portion 280 is fixed on the distal side as compared to the case where the cover portion 280 is fixed to the shaft portion 270. Therefore, after the distal end of the cover portion 280 passes through the hole of the living body tissue, the coarse pitch portion 281 easily extends in the axial direction by its own restoring force. For this reason, the cover portion 280 can favorably cover the needle portion 16. Besides, the cover portion 280 is located on the distal side relative to the curved portion 271. For this reason, the cover portion 280 does not need to slide outside the curved portion 271. Therefore, it is easy for the cover portion 280 to shrink and extend in the axial direction. For this reason, the cover portion 280 can smoothly expose the needle portion 16 and can smoothly accommodate the needle portion 16. Further, when the curved portion 271 of the shaft portion 270 protrudes from the dilator 40 and passes through the hole of the living body tissue, the curved portion 271 is restored into a curved shape. Therefore, the distal end of the guide wire 260 does not easily abut on the living body tissue. For this reason, according to the guide wire 260, erroneous puncture by the needle portion 16 can be restrained by the curved portion 271. In addition, the cover portion 280 is fixed to the puncture portion 15 on the distal side of the shaft portion 270. Therefore, it is easy to circulate a fluid in a through-hole 17 of the puncture portion 15 without being obstructed by the cover portion 280. As a result, a contrast medium and blood can be favorably circulated through the through-hole 17.

In addition, as in a thirteenth modification depicted in FIG. 22, a shaft portion 270 of a guide wire 290 may have a curved portion 271, and a cover portion 300 may have a second curved portion 302. Note that the parts having functions similar to those of the parts in the aforementioned embodiment are denoted by the same reference symbols as used above, and descriptions thereof are omitted.

The cover portion 300 includes a coarse pitch portion 301 having a long pitch distance of a helix, and a second curved portion 302 located on the distal side relative to the coarse pitch portion 301. The coarse pitch portion 301 is formed of a wire member 31 in a helical shape with a gap between adjacent loops thereof. The second curved portion 302 is formed from the wire member 31 in a helical shape and formed into a bent tubular shape as a whole. The cover portion 300 is fixed to a proximal portion of a puncture portion 15 by a fixing portion 303 located at the proximal end of the cover portion 300. Note that the proximal end of the cover portion 300 may be located on the proximal side relative to the fixing portion 303.

The guide wire 290 is accommodated in a dilator 40, with the curved portion 271 and the second curved portion 302 deformed into a linear shape. When the guide wire 290 is moved toward the distal side in the inside of the dilator 40, the distal end of the guide wire 290 comes into contact with the living body tissue. Therefore, a proximally directed force acts on the distal end of the cover portion 300 located at the most distal position of the guide wire 290. As a result, the gap between adjacent loops of the wire member 31 of the coarse pitch portion 301 is reduced, and the coarse pitch portion 301 shrinks in the axial direction. Consequently, the distal portion of the cover portion 300 is moved toward the proximal side, and a needle portion 16 is exposed from the cover portion 300. The needle portion 16 exposed from the cover portion 300 forms a hole in the living body tissue. When the distal end of the cover portion 300 passes through the hole of the living body tissue subsequentially to the needle portion 16, the coarse pitch portion 301 extends in the axial direction by its own restoring force. As a result, the cover portion 300 covers the needle portion 16.

The cover portion 300 in the thirteenth modification is fixed to the puncture portion 15. In other words, the cover portion 300 is fixed on the distal side as compared to the case where the cover portion 300 is fixed to a shaft portion 270. Therefore, after the distal end of the cover portion 300 passes through the hole of the living body tissue, it is easy for the coarse pitch portion 301 to extend in the axial direction by its own restoring force. For this reason, the cover portion 300 can favorably cover the needle portion 16. In addition, the cover portion 300 is located on the distal side relative to the curved portion 271. Therefore, it is unnecessary for the cover portion 300 to slide on the outside of the curved portion 271. For this reason, it is easy for the cover portion 300 to shrink and extend in the axial direction. Therefore, the cover portion 300 can smoothly expose the needle portion 16 and can smoothly accommodate the needle portion 16. Further, when the curved portion 271 of the shaft portion 270 and the second curved portion 302 of the cover portion 300 protrude from the dilator 40 and pass through the hole in the living body tissue, the curved portions are restored into their curved shapes. Therefore, the distal end of the guide wire 290 does not easily abut on the living body tissue. For this reason, according to the guide wire 290, erroneous puncture by the needle portion 16 can be restrained by the curved portion 271 and the second curved portion 302. The guide wire 290 can effectively restrain erroneous puncture by the needle portion 16 since both the shaft portion 270 and the cover portion 300 are curved. In addition, the cover portion 300 is fixed to the puncture portion 15 on the distal side of the shaft portion 270. Therefore, it is easy to circulate a fluid in a through-hole 17 without being obstructed by the cover portion 300. As a result, a contrast medium and blood can be favorably circulated through the through-hole 17.

Besides, as in a fourteenth modification depicted in FIG. 23, a shaft portion 270 of a guide wire 310 may have a curved portion 271, and a cover portion 320 may be rectilinear in shape. Note that the parts having functions similar to those of the parts in the aforementioned embodiment are denoted by the same reference symbols as used above, and descriptions thereof are omitted.

Th cover portion 320 accommodates a puncture portion 15 such that the puncture portion 15 can protrude. The cover portion 320 includes a coarse pitch portion 321 having a long pitch distance of a helix, and a proximal tapered portion 322. The coarse pitch portion 321 is formed of a wire member 31 in a helical shape with a gap between adjacent loops thereof. The proximal tapered portion 322 is located on the proximal side of the coarse pitch portion 321. The proximal tapered portion 322 is decreased in diameter in a tapered form toward the proximal side. The cover portion 320 is fixed to the shaft portion 270 by a fixing portion 323 located at the proximal end of the proximal tapered portion 322. Note that the proximal portion of the cover portion 320 may not be formed in a tapered shape.

The guide wire 310 is accommodated in a dilator 40, with the curved portion 271 in a rectilinearly deformed state. When the guide wire 310 is moved toward the distal side in the inside of the dilator 40, the distal end of the guide wire 310 comes into contact with the living body tissue. Therefore, a proximally directed force acts on the distal end of the cover portion 320 located at the most distal position of the guide wire 310. As a result, the gap between adjacent loops of the wire member 31 of the coarse pitch portion 321 is reduced, and the coarse pitch portion 321 shrinks in the axial direction. Consequently, a distal portion of the cover portion 320 is moved toward the proximal side, and a needle portion 16 is exposed from the cover portion 320. The needle portion 16 exposed from the cover portion 320 forms a hole in the living body tissue. When the distal end of the cover portion 320 passes through the hole in the living body tissue subsequentially to the needle portion 16, the coarse pitch portion 321 extends in the axial direction by its own restoring force. As a result, the cover portion 320 covers the needle portion 16.

The cover portion 320 in the fourteenth modification is fixed to the shaft portion 270. In other words, the cover portion 320 is fixed on the proximal side as compared to the case where the cover portion 320 is fixed to the puncture portion 15. Therefore, the cover portion 320 can be enlarged in the axial length, and a contraction/extension distance thereof can be set long. For this reason, the cover portion 320 can be formed to be long on the distal side relative to the needle portion 16. Therefore, the cover portion 320 can restrain unintentional exposure of the needle portion 16 and can cover the needle portion 16 safely and favorably. In addition, when the curved portion 271 of the shaft 270 protrudes from the dilator 40 and passes through the hole in the living body tissue, the curved portion 271 is restored into a curved shape. Therefore, the distal end of the guide wire 310 does not easily abut on the living body tissue. For this reason, according to the guide wire 310, erroneous puncture by the needle portion 16 can be restrained by the curved portion 271 of the shaft portion 270. Besides, the cover portion 320, by being fixed to the shaft portion 270, surrounds that part between the proximal end of the puncture portion 15 and the shaft portion 270 at which rigidity varies largely. Therefore, the cover portion 320 can reduce variation in the rigidity of the distal portion of the guide wire 310, and can easily transmit, to the needle portion 16, a force for pressing the guide wire 310 forward.

Note that the distal portion of the cover portion 320 in the fourteenth modification may be provided with a second curved portion similar to the distal portion of the cover portion 300 in the thirteenth modification.

In addition, as in a fifteenth modification depicted in FIG. 24, the angle of bending of a curved portion 422 provided in a shaft portion 420 of a guide wire 410 may be less than 90 degrees.

The guide wire 410 may include the shaft portion 420 having a curved portion 422, and a cover portion 330 having a second curved portion 335. Note that the parts having functions similar to those of the parts in the aforementioned embodiment are denoted by the same reference symbols as used above, and descriptions thereof are omitted.

The cover portion 330 includes a coarse pitch portion 331 having a long pitch distance of a helix, a dense pitch portion 332 having a short pitch distance of the helix, and a proximal tapered portion 333. The dense pitch portion 332 is located on the proximal side of the coarse pitch portion 331. The proximal tapered portion 333 is located on the proximal side of the dense pitch portion 332. The proximal tapered portion 333 is decreased in diameter in a tapered form toward the proximal side. The coarse pitch portion 331 can shrink in the axial direction of the guide wire 410. The dense pitch portion 332, by surrounding the shaft portion 420 decreasing in diameter, provides the guide wire 410 with a high pressing-in property. The cover portion 330 is fixed to the shaft portion 420 by a fixing portion 334 located at the proximal end of the proximal tapered portion 333. Note that a proximal portion of the cover portion 330 may not be formed in a tapered shape. The cover portion 330 has a second curved portion 335 in the dense pitch portion 332. The direction in which the second curved portion 335 is curved coincides with the direction in which the curved portion 422 is curved.

In the fifteenth modification, immediately after a puncture portion 15 penetrates the living body tissue (oval fossa O) and before the curved portion 422 penetrates the living body tissue, the cover portion 330 extends in the axial direction to automatically cover the puncture portion 15, as illustrated in FIG. 25(A). Therefore, the time for which the puncture portion 15 is in a bare state in the living body can be shortened, and safety is enhanced. In addition, as depicted in FIG. 25(B), after the curved portion 422 penetrates the living body tissue, a distal portion of the shaft portion 420 is automatically bent at an angle of less than 90 degrees. Therefore, in the case where the distal end of the cover portion 330 attaches to the living body tissue not to be punctured, such as a wall surface of a left atrium, a force can be relieved such that the puncture portion 15 will not be directed to the living body tissue not to be punctured. Besides, the puncture portion 15 can be prevented from being directed, for example, to a surface of the penetrated living body tissue on the left atrium side (atrial septum where the oval fossa O is present). As a result, damage to the atrial septum or the like by the puncture portion 15 can be restrained. In addition, since the shaft portion 420 is bent at an angle of less than 90 degrees, it is easy to adjust the direction of the distal end of the cover portion 330 by operating the guide wire 410 at hand. Therefore, after the living body tissue is punctured, the distal end of the cover portion 330 can be directed to any direction. For this reason, the guide wire 410 can enhance the function of making access to a target blood vessel in the manner of preceding other devices. Besides, the distal end of the curved portion 422 is located on the proximal side of the puncture portion 15. Therefore, the cover portion 330 can contract and extend in the axial direction without being influenced by the curvature of the curved portion 422. For this reason, the cover portion 330 can smoothly cover the puncture portion 15. The curved portion 422 may be a part of a portion ranging from the rectilinear portion of the shaft portion 420 to the distal end of the shaft portion 420. In that case, the curved portion 422 is interposed between rectilinear portions of the shaft portion 420.

Note that as illustrated in FIG. 26, the shaft portion 420 in the fifteenth modification may have a third curved portion 423 on the proximal side relative to the curved portion 422 and the second curved portion 335. The third curved portion 423 is located on the proximal side relative to the cover portion 330. The direction in which the third curved portion 423 is curved coincides with the direction in which the curved portion 422 and the second curved portion 335 are curved. The third curved portion 423 is provide at angle and position substantially coincident with those of the dilator bent portion 46 (see FIG. 1) of the dilator 40. The third curved portion 423 is provided, for example, at a position deviated by approximately 100 mm toward the proximal side from the distal end of the guide wire 410. Provided with the third curved portion 423 corresponding to the dilator bent portion 46, the guide wire 410 can effectively transmit a pressing-in force toward the distal side in the inside of the dilator 40. Therefore, the guide wire 410 may be used by being directly inserted in the dilator 40 without using the inserter 60.

In addition, as in a sixteenth modification depicted in FIG. 27, a guide wire 350 may include a shaft portion 360 having an easily plastically deformable curved portion 361, and a cover portion 370 having a second curved portion 374. Note that the parts having functions similar to those of the parts in the aforementioned embodiment are denoted by the same reference symbols as used above, and descriptions thereof are omitted.

The shaft portion 360 has the curved portion 361 which is easily plastically deformable. The curved portion 361 is provided at a part of a distal portion of the shaft portion 360. The curved portion 361 can be put into a state of being curved suitably according to a situation, by being curved as required by the operator. Note that the curved portion 361 may not be curved by the operator. The curved portion 361 can be formed, for example, by subjecting the whole part of a wire member of a superelastic alloy (shape memory alloy) such as NiTi to a heat treatment to impart superelasticity to the wire member and then partially annealing the wire member. Note that the position where the shaft portion 360 is provided with the curved portion 361 is not particularly limited. In addition, the cover portion 370 may partially have an easily plastically deformable curved portion.

The cover portion 370 includes a proximal tapered portion 375, a first coarse pitch portion 371 located on the distal side of the proximal tapered portion 375, a dense pitch portion 372 located on the distal side of the coarse pitch portion 371, and a second coarse pitch portion 373 located on the distal side of the dense pitch portion 372. The first coarse pitch portion 371 and the second coarse pitch portion 373 are formed of a wire member 31 in a helical shape with a gap between adjacent loops thereof. The proximal tapered portion 375 is decreased in diameter in a tapered form toward the proximal side from the first coarse pitch portion 371. The first coarse pitch portion 371 is located on the proximal side relative to a puncture portion 15. The dense pitch portion 372 has a shorter pitch distance of a helix than the first coarse pitch portion 371 and the second coarse pitch portion 373. The second coarse pitch portion 373 surrounds at least a part of the puncture portion 15. The cover portion 370 is fixed to the shaft portion 360 by a first fixing portion 377 located at the proximal end of the proximal tapered portion 375. The cover portion 370 is further fixed to a proximal portion of the puncture portion 15 by a second fixing portion 376 located at a proximal portion of the dense pitch portion 372. The second coarse pitch portion 373 has a second curved portion 374.

The guide wire 350 is used by plastically deforming the curved portion 361 as required by the operator such that the guide wire 350 is easily operated inside a body lumen. The guide wire 350 is accommodated in the dilator 40 in a state in which the curved portion 361 and the second curved portion 374 which are curved are elastically deformed into a rectilinear shape. When the guide wire 350 is moved toward the distal side in the inside of the dilator 40 and the distal end of the guide wire 350 comes into contact with the living body tissue, a proximally directed force acts on the distal end of the cover portion 370 located at the most distal position of the guide wire 350. As a result, the gap between adjacent loops of the wire member 31 of the second coarse pitch portion 373 is reduced, and the second coarse pitch portion 373 shrinks in the axial direction. Consequently, a distal portion of the cover portion 370 is moved toward the proximal side, and a needle portion 16 is exposed from the cover portion 370. The needle portion 16 exposed from the cover portion 370 forms a hole in the living body tissue. When the distal end of the cover portion 370 passes through the hole of the living body tissue subsequentially to the needle portion 16, the second coarse pitch portion 373 extends in the axial direction by its own restoring force. As a result, the cover portion 370 covers the needle portion 16. Note that since the second fixing portion 376 is provided on the distal side of the first coarse pitch portion 371, the first coarse pitch portion 371 does not shrink. Since the first coarse pitch portion 371 which does not shrink is provided in the cover portion 370 on the proximal side of the puncture portion 15, it is easy to circulate a fluid in the through-hole 17 via the wide gaps of the first coarse pitch portion 371. As a result, a contrast medium and blood can be favorably circulated through the through-hole 17. When the curved portion 361 and the second curved portion 374 protrude from the dilator 40 and pass through the hole in the living body tissue, the curved portions are restored into curved shapes. Therefore, the distal end of the guide wire 350 hardly abuts on the living body tissue. For this reason, according to the guide wire 350, erroneous puncture by the needle portion 16 can be restrained by the curved portion 361 and the second curved portion 374. The guide wire 350 can effectively restrain erroneous puncture by the needle portion 16 since both the curved portion 361 and the cover portion 370 are curved.

Besides, as in a seventeenth modification depicted in FIG. 28(A), a puncture portion 380 may be curved at a puncture curved portion 381. The direction in which the puncture curved portion 381 is curved coincides with the direction in which a second curved portion 374 of a cover portion 370 is curved, and coincides with the direction in which a sharp needle portion 16 is located in an inclined surface 18. Therefore, when the needle portion 16 is moved toward the distal side in the inside of the second curved portion 374 of the cover portion 370, the needle portion 16 can be restrained from making contact with an inner peripheral surface of the second curved portion 374. For this reason, the needle portion 16 can be restrained from unintentionally protruding from the second curved portion 374 to damage the living body tissue, so that safety is enhanced. In addition, with the puncture curved portion 381 provided, the whole length in the axial direction of the puncture portion 380 that can be moved in the inside of the second curved portion 374 is elongated. Therefore, a thick living body tissue can be punctured by the long puncture portion 380. Besides, with the puncture portion 380 elongated, the length from the second fixing portion 376 joined to the cover portion 370 by welding or the like to the needle portion 16 can be secured to be long. As a result, the length in the axial direction of the shrinkable second coarse pitch portion 373 of the cover portion 370 can be secured to be long. For this reason, the cover portion 370 can favorably accommodate the puncture portion 380, and safety is enhanced.

In addition, as in an eighteenth modification depicted in FIG. 28(B), a puncture portion 390 may have, at the proximal end thereof, a proximal inclined surface 391 which is inclined. The position of a projected portion 392 projected to the proximal side of the proximal inclined surface 391 overlaps with the position of an inclined surface 18 where a needle portion 16 is provided, in the axial direction. Therefore, an axially longest part (a part extending from the projected portion 392 to the needle portion 16) of the puncture portion 390 is located on the side to which a second curved portion 374 of a cover portion 370 is curved. For this reason, the overall length in the axial direction of the puncture portion 390 movable inside the second curved portion 374 is elongated. Therefore, a thick living body tissue can be punctured by the long puncture portion 390. Besides, with the puncture portion 390 elongated, the length from a second fixing portion 376 joined to the cover portion 370 by welding or the like to the needle portion 16 can be secured to be long. As a result, the cover portion 370 can secure a shrinkable length to be long and can accommodate the puncture portion 380 favorably.

Besides, as in a nineteenth modification depicted in FIG. 28(C), a puncture portion 400 may have, at a proximal-side end surface, a chamfered part 401 chamfered over 360 degrees. As a result, the overall length in the axial direction of the puncture portion 400 movable inside a second curved portion 374 is elongated. Therefore, a thick living body tissue can be punctured by the long puncture portion 400. In addition, with the puncture portion 400 elongated, the length from a second fixing portion 376 joined to a cover portion 370 by welding or the like to a needle portion 16 can be secured to be long. As a result, the cover portion 370 can secure a shrinkable length to be long and can accommodate the puncture portion 380 favorably.

In addition, as in twentieth modification depicted in FIG. 29, a guide wire 430 may have a structure curved as desired during used. Note that the parts having functions similar to those of the parts in the aforementioned embodiment are denoted by the same reference symbols as used above, and descriptions thereof are omitted.

A puncture portion 15 is fixed to a distal portion of a shaft portion 11 by a needle fixing portion 19. A cover portion 440 includes a coarse pitch portion 441 on the distal side and a dense pitch portion 442 on the proximal side. The cover portion 440 is fixed to a proximal portion of the puncture portion 15 by a second fixing portion 443 located in the vicinity of the boundary between the coarse pitch portion 441 and the dense pitch portion 442. The coarse pitch portion 441 is formed of a wire member 31 in a helical shape with a gap between adjacent loops thereof. The coarse pitch portion 441 surrounds the puncture portion 15 and extends to the distal side relative to the puncture portion 15. The dense pitch portion 442 is located on the proximal side of the coarse pitch portion 441. The dense pitch portion 442 is formed of the wire member 31 in a helical shape with no gap between adjacent loops thereof. The proximal end of the dense pitch portion 442 is located on the distal side relative to the proximal end of the shaft portion 11. A projected portion 444 projected radially outward is fixed to an outer peripheral surface of the proximal portion of the dense pitch portion 442. The projected portion 444 is, for example, a ring-shaped or tubular member. The projected portion 444 is fixed to the cover portion 440 in a non-detachable manner. Note that the projected portion 444 may be detachably fixed to the cover portion 440. That part of the shaft portion 11 which is on the proximal side relative to the projected portion 444 is a grip part 445 exposed without being covered with the cover portion 440.

The guide wire 430 is used in the state of being inserted in an assembly in which an outer sheath 50, a dilator 40, and an inserter 60 are combined. When the guide wire 430 is moved toward the distal side in the inside of the assembly and the distal end of the guide wire 430 comes into contact with the living body tissue, a proximally directed force acts on the distal end of the cover portion 440 located at the most distal position of the guide wire 430. As a result, the gap between adjacent loops of the wire member 31 of the coarse pitch portion 441 is reduced, and the coarse pitch portion 441 shrinks in the axial direction. Consequently, a distal portion of the cover portion 440 is moved toward the proximal side, and a needle portion 16 is exposed from the cover portion 440. The needle portion exposed from the cover portion 440 forms a hole in the living body tissue. When the distal end of the cover portion 440 passes through the hole in the living body tissue subsequentially to the needle portion 16, the coarse pitch portion 441 extends in the axial direction by its own restoring force, as illustrated in FIG. 29(A). As a result, the cover portion 440 covers the needle portion 16.

Further, when the shaft portion 11 is pressed forward, the projected portion 444 abuts on the inserter 60. As a result, distal movement of the projected portion 444 and the cover portion 440 fixed to the projected portion 444 is restricted. Further, when the grip part 445 is gripped and the shaft portion 11 is pressed toward the distal side, the shaft portion 11 is moved toward the distal side relative to the projected portion 444 and the cover portion 440 which are restricted in distal movement, as depicted in FIG. 30(B). When the shaft portion 11 is moved toward the distal side, a distally directed force acts on the puncture portion 15 fixed to the distal portion of the shaft portion 11 by a needle fixing portion 19. The puncture portion 15 is fixed to the cover portion 440 by a second fixing portion 443. Therefore, a proximally directed force acts on the distal portion of the cover portion 440 restricted in distal movement due to being fixed to the projected portion 444. A pulling force acts on the dense pitch portion 442, whereby a pitch distance of the dense pitch portion 442 is enlarged. Note that the pitch distance of the dense pitch portion 442 may not be enlarged. When a proximally directed force acts on the distal portion of the cover portion 440, the distal portion of the cover portion 440 that protrudes toward the distal side from the dilator 40 is curved in some direction. In this instance, the shaft portion 11 located inside the cover portion 440 is also curved together with the cover portion 440. Therefore, the guide wire 430 according to the twentieth modification can be curved as desired, by moving the shaft portion 11 relative to the cover portion 440. For this reason, according to the guide wire 430, erroneous puncture by the needle portion 16 can be restrained by an operation at hand. Note that the distal portion of the shaft portion 11 is fixed to the cover portion 440 through the puncture portion 15, however, the distal portion may be fixed directly to the cover portion 440.

In addition, the puncture portion may not be hollow but may be solid. Besides, the puncture portion may be a member which is non-continuous in the circumferential direction, and may have a C-shaped cross section. The puncture portion can be easily formed, for example, by press working of a plate material. The puncture portion may be fixed to the shaft portion by welding, adhesion, or the like, or may be integrally formed with the shaft portion.

The characteristics of each embodiment described above may be summarized as follows.

The guide wire 10 according to the first embodiment is provided with the curved portion 35 at a distal portion of the cover portion 30. The cover portion 30 includes the dense pitch portion 34 on the distal side, and the coarse pitch portion 33 on the proximal side. The cover portion 30 is fixed to the shaft portion 11 at the proximal end (see FIGS. 1 to 9).

The guide wire 80 according to the first modification is provided with the dense pitch portion 82 at a proximal portion of the cover portion 81, and the dense pitch portion 82 is formed with the circulation holes 84 (see FIG. 10). The guide wire 90 according to the second modification has the curved portion 92 which is a pipe-shaped member (see FIG. 11). The guide wire 110 according to the third modification is formed with the slits 113 in the cover portion 111 which is a pipe-shaped member (see FIG. 12). The cover portion 111 provided with the slits 113 can expand radially outward.

The guide wire 130 according to the fourth modification has the distal pipe 141, which is a circular pipe, on the distal side relative to the curved portion 35 of the cover portion 140 (see FIG. 13). The guide wires 150 according to the fifth and sixth modifications are formed with the inclined part 161 at the distal end of the cover portion 160 (see FIGS. 14 and 15).

The guide wire 170 according to the seventh modification has the first stopper 181 and the second stopper 182 (see FIG. 16). The first stopper 181 restricts proximal movement of the puncture portion 185. The second stopper 182 restricts distal movement of the puncture portion 185. A proximal portion of the puncture portion 185 has been subjected to spiral cutting. The guide wire 190 according to the eighth modification has the first stopper 204 and the second stopper 205 (see FIG. 17). The first stopper 204 restricts proximal movement of the puncture portion 15. The second stopper 205 restricts distal movement of the puncture portion 15.

The guide wires 210 and 220 according to the ninth and tenth modifications have protruding parts 212 and 231 protruding to the outer side relative to the inclined surface 18 of the puncture portion 15 (see FIGS. 18 and 19). The guide wire 240 according to the eleventh modification has the first markers 241 and 242 having an X-ray contrast property and the second marker 251 as a graduation at hand (see FIG. 20).

The guide wire 260 according to the twelfth modification has the shaft portion 270 formed with the curved portion 271, and the rectilinear cover portion 280 provided on the distal side relative to the curved portion 271 (see FIG. 21). The cover portion 280 is fixed to the puncture portion 15. The guide wire 290 according to the thirteenth modification has the shaft portion 270 formed with the curved portion 271, and the cover portion 300 provided on the distal side relative to the curved portion 271 and formed with the second curved portion 302 (see FIG. 22). The guide wire 310 according to the fourteenth modification has the shaft portion 270 formed with the curved portion 271, and the rectilinear cover portion 320 provided on the distal side relative to the curved portion 271 (see FIG. 23). The cover portion 320 is fixed not to the puncture portion 15 but to the shaft portion 270.

The guide wire 410 according to the fifteenth modification has the shaft portion 420 formed with the curved portion 422 curved at an angle of less than 90 degrees, and the cover portion 330 formed with the second curved portion 335 (see FIGS. 24 and 25). The second curved portion 335 surrounds the curved portion 422. The cover portion 330 is fixed to both the puncture portion 15 and the shaft portion 420. The shaft portion 420 may be provided with the third curved portion 423 corresponding to the dilator bent portion 46 (see FIG. 26).

The guide wire 350 according to the sixteenth modification has the shaft portion 360 provided with the curved portion 361 which is easily plastically deformable, and the cover portion 370 provided with the second curved portion 374 (see FIG. 27). The operator can curve the curved portion 361 as desired.

The guide wire according to the seventeenth modification has the puncture portion 380 formed with the puncture curved portion 381 (see FIG. 28 (A)). The guide wire according to the eighteenth modification has the puncture portion 390 formed with the proximal inclined surface 391 at the proximal end (see FIG. 28(B)). The guide wire according to the nineteenth modification has the puncture portion 390 formed with the chamfered part 401 at the proximal end (see FIG. 28(C)).

The guide wire 430 according to the twentieth modification has the structure in which the shaft portion 11 and the cover portion 440 can be curved as desired by pressing the shaft portion 11 forward (see FIGS. 29 and 30).

Note that the present application is based on Japanese Patent Application No. 2018-037626 filed on March 2, 2018.

### Description of Reference Symbols

1 Medical device
10, 80, 90, 110, 130, 150, 170, 190, 210, 220, 240, 260, 290, 310, 330, 350, 410, 430 Guide wire
11, 230, 250, 270, 360, 420 Shaft portion
12 Shaft proximal portion
13 Shaft decreasing diameter portion
14 Shaft distal portion
15, 185, 380, 390, 400 Puncture portion
16 Needle portion
17 Through-hole
17A Inner edge part
19, 211 Needle fixing portion
30, 81, 91, 111, 140, 160, 180, 200, 280, 300, 320, 370, 440 Cover portion
31 Wire member
34, 82, 202, 372, 442 Dense pitch portion
33, 72, 83, 201, 281, 301, 321, 441 Coarse pitch portion
35, 92, 115, 206, 271, 361, 374, 422 Curved portion
40 Dilator (Elongate body)
50 Outer sheath (Elongate body)
60 Inserter (Elongate body)
112 Distal cover portion
114 Proximal cover portion
141, 203 Distal pipe
161 Inclined part
181, 204 First stopper (Stopper)
182, 205 Second stopper (Stopper)
212, 231 Protruding part
241, 242 First marker (Marker)
251 Second marker
302, 335, 374 Second curved portion
371 First coarse pitch portion
373 Second coarse pitch portion
423 Third curved portion
O Oval fossa (Living body tissue)
L Left atrium
R Right atrium

## Claims

1. A guide wire (10, 80, 90, 110, 130, 150, 170, 190, 210, 220, 240, 260, 290, 310, 330, 350, 410, 430) for guiding a pipe-shaped elongate body (40) to be inserted in a living body, the guide wire (10) comprising:
a flexible elongate shaft portion (11, 230, 250, 270, 360, 420);
a puncture portion (15, 185, 380, 390, 400) that is disposed at a distal portion of the shaft portion (11, 230, 250, 270, 360, 420) and that is capable of forming a hole in living body tissue;
**characterised in that**,
it further comprises a cover portion (30, 81, 91, 111, 140, 160, 180, 200, 280, 300, 320. 370, 440) that is elastically deformable and that covers the puncture portion (15, 185, 380, 390, 400),
wherein the cover portion (30, 81, 91, 111, 140, 160, 180, 200, 280, 300, 320, 370, 440) is located on a distal portion of the shaft portion (11, 230, 250, 270, 360, 420) by a fixing portion (36), when the cover portion (30, 81, 91, 111, 140, 160, 180, 200, 280, 300, 320, 370, 440) is exposed from the elongate body (40), the cover portion (30, 81, 91, 111, 140, 160, 180, 200, 280, 300, 320, 370, 440) covers a distal end of the puncture portion (15, 185, 380, 390, 400), and at least one of the cover portion (30, 81, 91, 111, 140, 160, 180, 200, 280, 300, 320, 370, 440) and the shaft portion (11, 230, 250, 270, 360, 420) is bent.

2. The guide wire (10) according to claim 1,
wherein at least one of the cover portion (30) and the shaft portion (11) is deformable into a rectilinear shape by being accommodated in the elongate body (40).

3. The guide wire (10) according to claim 1 or 2,
wherein the cover portion (30) is fixed to the shaft portion (11),
the cover portion (30), in a state of being accommodated inside the elongate body (40), receives a proximally directed force at a distal end thereof to be elastically shrunk, thereby exposing the puncture portion (15), and
the cover portion (30), in a state of being located outside the elongate body (40), keeps a state of accommodating the puncture portion (15) while covering the puncture portion (15).

4. The guide wire (10) according to any one of claims 1 to 3,
wherein at least one of the cover portion (30) and the shaft portion (11) has a curved portion (35, 92, 115, 206, 271, 361, 374, 422) which is bent in a natural state.

5. The guide wire (10) according to claim 4,
wherein a proximal end of the curved portion (35) is located on a distal side of the puncture portion (15).

6. The guide wire (10) according to claim 4,
wherein a distal end of the curved portion (35) is located on a proximal side of the puncture portion (15).

7. The guide wire (10) according to any one of claims 1 to 6,
wherein the puncture portion (15) has a through-hole (17) penetrating in an axial direction.

8. The guide wire (10) according to any one of claims 1 to 7,
wherein the cover portion (30) is a helical member, and at least part of the cover portion (30) has a gap between adjacent loops of a wire member (31) aligned while forming a helix.

9. The guide wire (10) according to claim 8,
wherein the cover portion (30) includes a coarse pitch portion (33, 72, 83, 201, 281, 301, 321, 441) that has a gap between adjacent loops of the wire member (31) aligned while forming a helix, and a dense pitch portion (34, 82, 202, 372, 442) that is located on a proximal side or a distal side of the coarse pitch portion (33) and that is shorter than the coarse pitch portion (33) in pitch distance of the wire member (31) aligned while forming a helix.

10. The guide wire (10) according to any one of claims 1 to 9,
wherein a distal portion of the puncture portion (15) has an inclined surface (18) inclined relative to a center axis of the puncture portion (15),
a sharp needle portion (16) of the puncture portion (15) is located at a distal end of the inclined surface (18), and
a direction in which at least one of the cover portion (30) and the shaft portion (11) is bent coincides with a direction in which the needle portion (16) is located at the inclined surface (18).

11. The guide wire (10) according to any one of claims 1 to 10,
wherein the cover portion (30) has a stopper (181, 182, 204, 205) for limiting an axial movement of the puncture portion (15) relative to the cover portion (30).

12. The guide wire (10) according to any one of claims 1 to 11,
wherein the puncture portion (15) includes an inclined surface (18) provided at a distal portion thereof and inclined relative to the center axis of the puncture portion (15), and a through-hole (17) penetrating from the inclined surface (18) toward the proximal side,
the shaft portion (11) has a needle fixing portion (19, 211) fixed to an inner peripheral surface of the through-hole (17), and
at least part of the needle fixing portion (19) protrudes to outside of a surface at which a ring-shaped inner edge part (17A) of an opening of the through-hole (17) in the inclined surface (18) is located.

13. A medical device (1) for forming a hole in living body tissue of a living body, the medical device (1) comprising:
a pipe-shaped elongate body (40) configured to be inserted in the living body; and
a guide wire (10) according to any one of the preceding claims.

## Patentansprüche

1. Führungsdraht (10, 80, 90, 110, 130, 150, 170, 190, 210, 220, 240, 260, 290, 310, 330, 350, 410, 430) zum Führen eines rohrförmigen, langgestreckten Körpers (40), der in einen lebenden Körper eingeführt werden soll, wobei der Führungsdraht (10) umfasst:
einen flexiblen langgestreckten Schaftabschnitt (11, 230, 250, 270, 360, 420);
einen Punktionsabschnitt (15, 185, 380, 390, 400), der an einem distalen Abschnitt des Schaftabschnitts (11, 230, 250, 270, 360, 420) angeordnet ist und der in der Lage ist, ein Loch in einem lebenden Körpergewebe zu bilden;
**dadurch gekennzeichnet, dass** er ferner einen Abdeckabschnitt (30, 81, 91, 111, 140, 160, 180, 200, 280, 300, 320, 370, 440) umfasst, der elastisch verformbar ist und den Punktionsabschnitt (15, 185, 380, 390, 400) abdeckt,
wobei der Abdeckabschnitt (30, 81, 91, 111, 140, 160, 180, 200, 280, 300, 320, 370, 440) mittels eines Befestigungsabschnitts (36) an einem distalen Abschnitt des Schaftabschnitts (11, 230, 250, 270, 360, 420) angeordnet ist, wenn der Abdeckabschnitt (30, 81, 91, 111, 140, 160, 180, 200, 280, 300, 320, 370, 440) von dem langgestreckten Körper (40) freigelegt ist, der Abdeckabschnitt (30, 81, 91, 111, 140, 160, 180, 200, 280, 300, 320, 370, 440) ein distales Ende des Punktionsabschnitts (15, 185, 380, 390, 400) abdeckt, und mindestens einer von dem Abdeckabschnitt (30, 81, 91, 111, 140, 160, 180, 200, 280, 300, 320, 370, 440) und dem Schaftabschnitt (11, 230, 250, 270, 360, 420) gebogen ist.

2. Führungsdraht (10) nach Anspruch 1,
wobei mindestens einer von dem Abdeckungsabschnitt (30) und dem Schaftabschnitt (11) in eine geradlinige Form verformbar ist, indem er in den langgestreckten Körper (40) aufgenommen wird.

3. Führungsdraht (10) nach Anspruch 1 oder 2,
wobei der Abdeckabschnitt (30) an dem Schaftabschnitt (11) befestigt ist,
der Abdeckabschnitt (30) in einem Zustand, in dem er in dem langgestreckten Körper (40) aufgenommen ist, an seinem distalen Ende davon eine proximal gerichtete Kraft aufnimmt, um elastisch zusammengezogen zu werden, wodurch der Punktionsabschnitt (15) freigelegt wird, und
der Abdeckabschnitt (30) in einem Zustand, in dem er außerhalb des langgestreckten Körpers (40) angeordnet ist, einen Zustand beibehält, in dem er den Punktionsabschnitt (15) aufnimmt, während er den Punktionsabschnitt (15) abdeckt.

4. Führungsdraht (10) nach einem der Ansprüche 1 bis 3,
wobei mindestens einer von dem Abdeckabschnitt (30) und dem Schaftabschnitt (11) einen gekrümmten Abschnitt (35, 92, 115, 206, 271, 361, 374, 422) aufweist, der in einem natürlichen Zustand gebogen ist.

5. Führungsdraht (10) nach Anspruch 4,
wobei ein proximales Ende des gekrümmten Abschnitts (35) auf einer distalen Seite des Punktionsabschnitts (15) angeordnet ist.

6. Führungsdraht (10) nach Anspruch 4,
wobei ein distales Ende des gekrümmten Abschnitts (35) auf einer proximalen Seite des Punktionsabschnitts (15) angeordnet ist.

7. Führungsdraht (10) nach einem der Ansprüche 1 bis 6,
wobei der Punktionsabschnitt (15) ein Durchgangsloch (17) aufweist, das in einer axialen Richtung hindurchdringt.

8. Führungsdraht (10) nach einem der Ansprüche 1 bis 7,
wobei der Abdeckabschnitt (30) ein spiralförmiges Element ist und mindestens ein Teil des Abdeckabschnitts (30) einen Spalt zwischen benachbarten Windungen eines Drahtelements (31) aufweist, die unter Bildung einer Spirale ausgerichtet sind.

9. Führungsdraht (10) nach Anspruch 8,
wobei der Abdeckabschnitt (30) einen Abschnitt mit weitem Teilungsabstand (33, 72, 83, 201, 281, 301, 321, 441) umfasst, der einen Spalt zwischen benachbarten Windungen des Drahtelements (31) aufweist, die unter Bildung einer Spirale ausgerichtet sind, und einen Abschnitt mit engem Teilungsabstand (34, 82, 202, 372, 442) umfasst, welcher auf einer proximalen Seite oder einer distalen Seite des Abschnitts mit weitem Teilungsabstand (33) angeordnet ist, und der in Bezug auf den Teilungsabstand der Drahtelemente (31), die unter Bildung einer Spirale ausgerichtet sind, kürzer ist als der Abschnitt mit weitem Teilungsabstand (33).

10. Führungsdraht (10) nach einem der Ansprüche 1 bis 9,
wobei ein distaler Abschnitt des Punktionsabschnitts (15) eine geneigte Fläche (18) aufweist, die relativ zu einer Mittelachse des Punktionsabschnitts (15) geneigt ist,
ein scharfer Nadelabschnitt (16) des Punktionsabschnitts (15) an einem distalen Ende der geneigten Fläche (18) angeordnet ist, und
eine Richtung, in der mindestens einer von dem Abdeckabschnitt (30) und dem Schaftabschnitt (11) gebogen ist, mit einer Richtung übereinstimmt, in welcher der Nadelabschnitt (16) an der geneigten Fläche (18) angeordnet ist.

11. Führungsdraht (10) nach einem der Ansprüche 1 bis 10,
wobei der Abdeckabschnitt (30) einen Anschlag (181, 182, 204, 205) aufweist, um eine axiale Bewegung des Punktionsabschnitts (15) relativ zu dem Abdeckabschnitt (30) einzuschränken.

12. Führungsdraht (10) nach einem der Ansprüche 1 bis 11,
wobei der Punktionsabschnitt (15) eine geneigte Fläche (18), welche an einem distalen Abschnitt davon vorgesehen ist, und relativ zu der Mittelachse des Punktionsabschnitts (15) geneigt ist, und ein Durchgangsloch (17) aufweist, welches von der geneigten Fläche (18) in Richtung zu der proximalen Seite hindurchdringt,
wobei der Schaftabschnitt (11) einen Nadelbefestigungsabschnitt (19, 211) aufweist, der an einer inneren Umfangsfläche des Durchgangslochs (17) befestigt ist, und
mindestens ein Teil des Nadelbefestigungsabschnitts (19) zur Außenseite einer Fläche hervorsteht, an der ein ringförmiger innerer Randabschnitt (17A) von einer Öffnung des Durchgangslochs (17) in der geneigten Fläche (18) angeordnet ist.

13. Medizinische Vorrichtung (1) zum Bilden eines Lochs in lebendem Körpergewebe eines lebenden Körpers, wobei die medizinische Vorrichtung (1) umfasst:
einen rohrförmigen, langgestreckten Körper (40), der so ausgebildet ist, dass er in den lebenden Körper eingeführt werden kann; und
einen Führungsdraht (10) nach einem der vorhergehenden Ansprüche.

## Revendications

1. Fil guide (10, 80, 90, 110, 130, 150, 170, 190, 210, 220, 240, 260, 290, 310, 330, 350, 410, 430) pour guider un corps allongé en forme de tuyau (40) à insérer dans un corps vivant, le fil guide (10) comprenant :
une partie tige allongée souple (11, 230, 250, 270, 360, 420) ;
une partie de perforation (15, 185, 380, 390, 400) qui est disposée au niveau d'une partie distale de la partie tige (11, 230, 250, 270, 360, 420) et qui est capable de former un trou dans un tissu de corps vivant ;
**caractérisé en ce que**,
il comprend en outre
une partie de recouvrement (30, 81, 91, 111, 140, 160, 180, 200, 280, 300, 320, 370, 440) qui est élastiquement déformable et qui recouvre la partie de perforation (15, 185, 380, 390, 400),
dans lequel la partie de recouvrement (30, 81, 91, 111, 140, 160, 180, 200, 280, 300, 320, 370, 440) est positionnée sur une partie distale de la partie tige (11, 230, 250, 270, 360, 420) au moyen d'une partie de fixation (36), lorsque la partie de recouvrement (30, 81, 91, 111, 140, 160, 180, 200, 280, 300, 320, 370, 440) est exposée à partir du corps allongé (40), la partie de recouvrement (30, 81, 91, 111, 140, 160, 180, 200, 280, 300, 320, 370, 440) recouvre une extrémité distale de la partie de perforation (15, 185, 380, 390, 400), et au moins l'une de la partie de recouvrement (30, 81, 91, 111, 140, 160, 180, 200, 280, 300, 320, 370, 440) et de la partie tige (11, 230, 250, 270, 360, 420) est pliée.

2. Fil guide (10) selon la revendication 1,
dans lequel au moins l'une de la partie de recouvrement (30) et de la partie tige (11) peut être déformée en une forme rectiligne en étant logée dans le corps allongé (40)

3. Fil guide (10) selon la revendication 1 ou 2,
dans lequel la partie de recouvrement (30) est fixée à la partie tige (11),
la partie de recouvrement (30), dans un état où elle est logée à l'intérieur du corps allongé (40), reçoit une force dirigée proximalement au niveau d'une extrémité distale de celle-ci pour être rétrécie élastiquement, exposant ainsi la partie de perforation (15), et
la partie de recouvrement (30), dans un état où elle est positionnée à l'extérieur du corps allongé (40), maintient un état de logement de la partie de perforation (15) tout en recouvrant la partie de perforation (15).

4. Fil guide (10) selon l'une quelconque des revendications 1 à 3,
dans lequel au moins l'une de la partie de recouvrement (30) et de la partie tige (11) présente une partie incurvée (35, 92, 115, 206, 271, 361, 374, 422) qui est pliée dans un état naturel.

5. Fil guide (10) selon la revendication 4,
dans lequel une extrémité proximale de la partie incurvée (35) est positionnée sur un côté distal de la partie de perforation (15).

6. Fil guide (10) selon la revendication 4,
dans lequel une extrémité distale de la partie incurvée (35) est positionnée sur un côté proximal de la partie de perforation (15).

7. Fil guide (10) selon l'une quelconque des revendications 1 à 6,
dans lequel la partie de perforation (15) présente un trou traversant (17) pénétrant dans une direction axiale.

8. Fil guide (10) selon l'une quelconque des revendications 1 à 7,
dans lequel la partie de recouvrement (30) est un organe hélicoïdal, et au moins une partie de la partie de recouvrement (30) présente un espace entre des boucles adjacentes d'un organe de fil (31) aligné tout en formant une hélice.

9. Fil guide (10) selon la revendication 8,
dans lequel la partie de recouvrement (30) comporte une partie de pas grossier (33, 72, 83, 201, 281, 301, 321, 441) qui présente un espace entre des boucles adjacentes de l'organe de fil (31) aligné tout en formant une hélice, et une partie de pas dense (34, 82, 202, 372, 442) qui est positionnée sur un côté proximal ou un côté distal de la partie de pas grossier (33) et qui présente une distance de pas plus courte que la partie de pas grossier (33) par rapport à l'organe de fil (31) aligné tout en formant une hélice.

10. Fil guide (10) selon l'une quelconque des revendications 1 à 9,
dans lequel une partie distale de la partie de perforation (15) présente une surface inclinée (18) qui est inclinée par rapport à un axe central de la partie de perforation (15),
une partie aiguille pointue (16) de la partie de perforation (15) est positionnée à une extrémité distale de la surface inclinée (18), et
une direction dans laquelle au moins l'une de la partie de recouvrement (30) et de la partie tige (11) est pliée coïncide avec une direction dans laquelle la partie aiguille (16) est positionnée au niveau de la surface inclinée (18).

11. Fil guide (10) selon l'une quelconque des revendications 1 à 10,
dans lequel la partie de recouvrement (30) présente une butée (181, 182, 204, 205) pour limiter un mouvement axial de la partie de perforation (15) par rapport à la partie de recouvrement (30).

12. Fil guide (10) selon l'une quelconque des revendications 1 à 11,
dans lequel la partie de perforation (15) comporte une surface inclinée (18) prévue au niveau d'une partie distale de celle-ci et inclinée par rapport à l'axe central de la partie de perforation (15), et un trou traversant (17) pénétrant à partir de la surface inclinée (18) vers le côté proximal,
la partie tige (11) présente une partie de fixation d'aiguille (19, 211) fixée à une surface périphérique interne du trou traversant (17), et
au moins une partie de la partie de fixation d'aiguille (19) fait saillie vers l'extérieur d'une surface au niveau de laquelle une partie de bord interne en forme d'anneau (17A) d'une ouverture du trou traversant (17) dans la surface inclinée (18) est positionnée.

13. Dispositif médical (1) pour former un trou dans un tissu de corps vivant d'un corps vivant, le dispositif médical (1) comprenant :
un corps allongé en forme de tuyau (40) configuré pour être inséré dans le corps vivant ; et
un fil guide (10) selon l'une quelconque des revendications précédentes.
